# EUROPEAN PATENT APPLICATION

(11) **EP 4 834 336 A1**
(43) Date of publication of application: **04.11.2026**
(21) Application number: 24913696.1
(22) Date of filing: 27.12.2024
(51) Int. Cl.: A61K 9/51, A61K 9/1272, A61K 31/7088, A61K 48/00

(54) **NANOPARTICLE COMPOSITION FOR DRUG DELIVERY**

(30) Priority: 29.12.2023 KR 20230197126
(71) Applicant: SAMYANG BIOPHARM CORPORATION, Seongnam-si Gyeonggi-do 13488 (KR)
(72) Inventor: YOO, Seung Ju, Seoul 07943 (KR); LEE, So Jin, Seongnam-si Gyeonggi-do 13479 (KR); NAM, Joung Pyo, Seoul 02471 (KR); PARK, Jong Min, Hwaseong-si Gyeonggi-do 18484 (KR)
(74) Representative: Gleiss Große Schrell und Partner mbB
(86) International application number: PCT/KR2024/021314
(87) International publication number: WO 2025/143892

(57) **Abstract**

The present invention relates to a composition for drug delivery, and a preparation method therefor, and, more specifically, to a composition for drug delivery, and a preparation method therefor, the composition having a form such that a drug is encapsulated inside a nanoparticle structure formed by a polymer and a cationic lipid with a specific structure.

## Description

### TECHNICAL FIELD

The present invention relates to a composition for drug delivery and a method for preparing the same, and more specifically, a composition for drug delivery which is in a form where a drug is encapsulated within a nanoparticle structure formed by a polymer and a cationic lipid having a specific structure, and a method for preparing the same.

### BACKGROUND ART

In therapies using anionic drugs including nucleic acid, technologies for safe and efficient drug delivery have been researched for a long time, and various carriers and techniques for delivery have been developed. Carriers are mainly divided into viral carriers utilizing adenovirus, retrovirus or the like, and non-viral carriers utilizing cationic lipid, cationic polymer or the like. Viral carriers are known as being exposed to risks such as non-specific immune response, etc. Thus, recent researches proceed in the direction to improve such disadvantages by using non-viral carriers. Although non-viral carriers are less efficient in comparison with viral carriers, they have advantages of fewer side effects in terms of *in vivo* safety.

The representative non-viral carriers for delivering nucleic acid material are a complex of cationic lipid and nucleic acid (lipoplex) and a complex of polycationic polymer and nucleic acid (polyplex). Such a cationic lipid or polycationic polymer stabilizes anionic drug by forming a complex through electrostatic interaction with the anionic drug and increases intracellular delivery, and for these reasons, various researches thereof have been conducted. However, when they are administered intravenously in an amount required to obtain a sufficient effect, severe toxicity is caused, although less toxic than viral carriers, resulting in that they are unsuitable for use in pharmaceutical products. Accordingly, there is a need to develop anionic drug delivery technology that is stable *in vivo* and capable of intracellular delivery to obtain sufficient effects, while reducing toxicity by minimizing the use of cationic polymer or cationic lipid that may cause toxicity.

Various anionic drug delivery compositions and preparation methods thereof have been disclosed, wherein a complex is formed by electrostatic interaction between nucleic acid and cationic lipid, and said complex is encapsulated within a nanoparticle structure of amphiphilic block copolymer. For example, Korean Laid-open Patent Publication No. 10-2017-0032858 discloses a composition for delivering an anionic drug, comprising the anionic drug as an active ingredient; a cationic compound; an amphiphilic block copolymer; and a salt of polylactic acid, wherein the anionic drug forms a complex with the cationic compound by electrostatic interaction, and the formed complex is encapsulated within the nanoparticle structure formed by the amphiphilic block copolymer and the salt of polylactic acid, and a method for preparing the same. However, including those disclosed in the above patent publication, the existing nanoparticle drug delivery systems still lack the efficiency to deliver drugs such as nucleic acid, polypeptide, or virus (especially mRNA) into the body.

### CONTENTS OF THE INVENTION

### PROBLEMS TO BE SOLVED

The purpose of the present invention is to provide a composition for drug delivery having significantly improved *in vivo* delivery efficiency of drugs such as nucleic acid, polypeptide, or virus (especially mRNA), as compared with previously known nanoparticle drug delivery systems, and a method for preparing the same.

### TECHNICAL MEANS

The first aspect of the present invention provides a composition for drug delivery comprising: effective ingredient selected from nucleic acid, polypeptide, virus or combination thereof; a cationic lipid having a structure represented by the following Formula 1; and lipid-polymer, amphiphilic block copolymer, or a combination thereof: 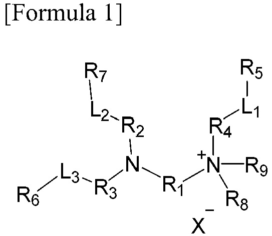 wherein, in the above Formula 1,
R₁ is a substituted or unsubstituted alkylene group, alkenylene group or alkynylene group,
each of R₂, R₃ and R₄ is independently a substituted or unsubstituted alkylene group, alkenylene group or alkynylene group,
each of R₅, R₆ and R₇ is independently a substituted or unsubstituted, saturated or unsaturated monovalent hydrocarbon group,
each of R₈ and R₉ is independently a substituted or unsubstituted alkyl group, alkenyl group, alkynyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, alkynylene, arylene, and heteroarylene, wherein L' is a direct bond, alkylene, alkenylene or alkynylene, and each R' is independently selected from the group consisting of hydrogen atom, alkyl, alkenyl and alkynyl,
n is 0 or 1, and
X⁻ is a pharmaceutically acceptable monovalent anion.

The second aspect of the present invention provides a method for preparing a composition for drug delivery, comprising the steps of: (a) preparing a solution in which a cationic lipid represented by the above Formula 1; and lipid-polymer, amphiphilic block copolymer, or a mixture thereof; are dissolved in a water-miscible organic solvent; and (b) to the solution prepared in step (a), adding effective ingredient selected from nucleic acid, polypeptide, virus, or combination thereof, and mixing them.

### EFFECT OF THE INVENTION

The composition for drug delivery according to the present invention can significantly improve *in vivo* delivery efficiency of drugs such as nucleic acid, polypeptide, or virus (especially mRNA), as compared with previously known nanoparticle drug delivery systems.

### BRIEF EXPLANATION OF THE DRAWINGS

Figure 1 is a reaction scheme for the synthesis procedure of Formula A compound conducted in Preparation Example 1.
Figure 2 is a reaction scheme for the synthesis procedure of Formula B compound conducted in Preparation Example 2.
Figure 3 is a reaction scheme for the synthesis procedure of Formula C compound conducted in Preparation Example 3.
Figure 4 is a reaction scheme for the synthesis procedures of Formulas D and E compounds conducted in Preparation Example 4.
Figure 5 is a reaction scheme for the synthesis procedure of Formula F compound conducted in Preparation Example 5.
Figure 6 is a reaction scheme for the synthesis procedure of Formula G compound conducted in Preparation Example 6.
Figure 7 is a reaction scheme for the synthesis procedure of Formula H compound conducted in Preparation Example 7.

### CONCRETE MODE FOR CARRYING OUT THE INVENTION

The present invention will be explained in detail below.

### Effective ingredient

The effective ingredient comprised in the composition for drug delivery of the present invention is selected from nucleic acid, polypeptide, virus, or combination thereof.

The "nucleic acid" may be, for example, DNA, RNA, siRNA, shRNA, miRNA, mRNA, aptamer, antisense oligonucleotide, or a combination thereof, but it is not limited thereto.

The "polypeptide" may mean a protein having activity in the body such as antibody or fragment thereof, cytokine, hormone or analog thereof, or a protein that can be recognized as antigen through a series of processes in the body, including polypeptide sequence of antigen, analog or precursor thereof.

The "virus" may be an oncolytic virus and, for example, may be one or more selected from the group consisting of adenovirus, AAV, vaccinia virus, herpes simplex virus (HSV), and vesicular stomatitis virus (VSV). In an embodiment, the oncolytic virus is an adenovirus. The adenovirus used in an embodiment of the present invention contains a luciferase gene, which can be confirmed through imaging.

The virus can express various types of therapeutic genes within the body of an individual and is not limited to specific molecular weight, protein, bioactivity or therapeutic field. The prophylactic virus can induce immunity within the body of an individual against a target disease. A composition containing a prophylactic virus to disease has the advantage of reducing immunity induction by the virus itself, capability of designating or expanding target cells, and reducing the hyperimmune response to the virus upon re-administration, thereby enabling effective effects to be obtained through multiple inoculations.

In an embodiment, the effective ingredient is mRNA (messenger RNA).

The mRNA may be chemically modified in its backbone, sugar, or base, or may be terminally modified, for purposes such as increasing stability in the bloodstream or reducing an immune response.

In an embodiment, the amount of the effective ingredient may be, based on the dry weight of the total composition, 0.05 wt% or more, 0.1 wt% or more, 0.2 wt% or more, 0.3 wt% or more, 0.4 wt% or more, or 0.5 wt% or more, and it may also be 10 wt% or less, 9 wt% or less, 8 wt% or less, 7 wt% or less, 6 wt% or less, 5 wt% or less, 4 wt% or less, or 3 wt% or less. If the amount of the effective ingredient is too less, the amount of delivery carrier becomes too much as compared with the drug, and thus there may be a side effect due to the delivery carrier. To the contrary, if the amount of the effective ingredient is too much, the amount of drug not encapsulated in nanoparticles becomes too much, and thus the efficiency decreases.

### Cationic lipid

The cationic lipid comprised in the nanoparticle composition of the present invention is has a structure represented by the following Formula 1: 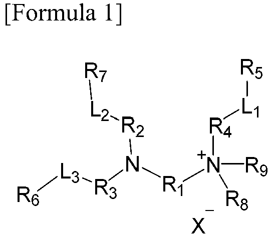 wherein, in the above Formula 1,
R₁ is a substituted or unsubstituted alkylene group, alkenylene group or alkynylene group,
each of R₂, R₃ and R₄ is independently a substituted or unsubstituted alkylene group, alkenylene group or alkynylene group,
each of R₅, R₆ and R₇ is independently a substituted or unsubstituted, saturated or unsaturated monovalent hydrocarbon group,
each of R₈ and R₉ is independently a substituted or unsubstituted alkyl group, alkenyl group, alkynyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, alkynylene, arylene, and heteroarylene, wherein L' is a direct bond, alkylene, alkenylene or alkynylene, and each R' is independently selected from the group consisting of hydrogen atom, alkyl, alkenyl and alkynyl,
n is 0 or 1, and
X⁻ is a pharmaceutically acceptable monovalent anion.

As used herein, the expression "substituted or unsubstituted" for any group means that, unless specified otherwise, the group is not substituted, or is substituted with one or more substituents selected from -OH, halogen atom, C₁₋₆ alkyl group, C₁₋₆ alkoxy group, C₁₋₆ halogenated alkyl group, C₁₋₆ halogenated alkoxy group, C₃₋₂₀ cycloalkyl group, C₃₋₂₀ heterocycloalkyl group, C₆₋₂₀ aryl group or C₃₋₂₀ heteroaryl group.

As used herein, the expression "hetero-" for any group (e.g., heteroaryl, heterocycloalkyl, etc.) means that, unless specified otherwise, the group has one or more (e.g., 1 to 3) heteroatoms selected from N, O and S.

As used herein, "monovalent hydrocarbon group" may be branched or unbranched, cyclic or acyclic, or aromatic.

As used herein, each of "alkyl," "alkenyl," "alkynyl," "alkylene," "alkenylene," and "alkynylene" may independently be branched or unbranched, or cyclic or acyclic.

According to an embodiment of the present invention,
R₁ may be a substituted or unsubstituted C₁₋₆ alkylene group, C₂₋₆ alkenylene group or C₂₋₆ alkynylene group,
each of R₂, R₃ and R₄ may be independently a substituted or unsubstituted C₃₋₁₂ alkylene group, C₃₋₁₂ alkenylene group or C₃₋₁₂ alkynylene group,
each of R₅, R₆ and R₇ may be independently a substituted or unsubstituted, saturated or unsaturated monovalent C₃₋₂₀ hydrocarbon group,
each of R₈ and R₉ may be independently a substituted or unsubstituted C₁₋₆ alkyl group, C₂₋₆ alkenyl group, C₂₋₆ alkynyl group or C₃₋₆ carbocyclic group, or may be independently -R₁₀-(L₄)ₙ-R₁₁,
each R₁₀ may be independently a substituted or unsubstituted C₃₋₁₂ alkylene group,
each R₁₁ may be independently a substituted or unsubstituted, saturated or unsaturated monovalent C₃₋₂₀ hydrocarbon group,
each of L₁, L₂, L₃ and L₄ may be independently selected from the group consisting of - C(O)O-, -OC(O)-, -OC(O)-L'-C(O)O-, -C(O)N(R')-, -N(R')C(O)-, -C(O)-, -C(S)-, -C(S)S-, - SC(S)-, -CH(OH)-, -P(O)(OR')O-, -S(O)₂-, -S-S-, C₂₋₆ alkenylene, C₂₋₆ alkynylene, C₆₋₂₀ arylene, and C₃₋₂₀ heteroarylene, wherein L' may be a direct bond, C₁₋₁₃ alkylene, C₂₋₁₃ alkenylene or C₂₋₁₃ alkynylene, and each R' may be independently selected from the group consisting of hydrogen atom, C₁₋₁₈ alkyl, C₂₋₁₈ alkenyl and C₂₋₁₈ alkynyl,
n is 0 or 1, and
X⁻ may be a pharmaceutically acceptable monovalent anion of inorganic acid or organic acid.

More specifically,
R₁ may be a substituted or unsubstituted C₃₋₄ alkylene group, C₃₋₄ alkenylene group or C₃₋₄ alkynylene group,
each of R₂, R₃ and R₄ may be independently a substituted or unsubstituted C₆₋₈ alkylene group, C₆₋₈ alkenylene group or C₆₋₈ alkynylene group,
each of R₅, R₆ and R₇ may be independently a substituted or unsubstituted, saturated or unsaturated monovalent C₅₋₁₅ hydrocarbon group,
each of R₈ and R₉ may be independently a substituted or unsubstituted C₁₋₂ alkyl group, C₂₋₃ alkenyl group or C₂₋₃ alkynyl group, or may be independently -R₁₀-(L₄)ₙ-R₁₁,
each R₁₀ may be independently a substituted or unsubstituted C₆₋₈ alkylene group,
each R₁₁ may be independently a substituted or unsubstituted, saturated or unsaturated monovalent C₅₋₁₅ hydrocarbon group,
each of L₁, L₂, L₃ and L₄ may be independently selected from the group consisting of - C(O)O-, -OC(O)-, -C(O)N(R')-, -N(R')C(O)-, -P(O)(OR')O-, -S-S-, C₂₋₅ alkenylene and C₂₋₅ alkynylene, wherein each R' may be independently selected from the group consisting of hydrogen atom, C₁₋₆ alkyl, C₂₋₆ alkenyl and C₂₋₆ alkynyl,
n is 0 or 1, and
X⁻ may be halide (F⁻, Cl⁻, Br⁻, I⁻), nitrate anion (NO₃⁻), benzoate anion (C₆H₅COO⁻), methanesulfonate anion, acetate anion (CH₃COO⁻) (i.e., AcO⁻), or trihaloacetate anion (CF₃COO⁻).

Even more specifically,
R₁ may be a substituted or unsubstituted C₃₋₄ alkylene group,
each of R₂, R₃ and R₄ may be independently a substituted or unsubstituted C₆₋₈ alkylene group,
each of R₅, R₆ and R₇ may be independently a substituted or unsubstituted, saturated or unsaturated monovalent C₅₋₁₅ hydrocarbon group,
each of R₈ and R₉ may be independently a substituted or unsubstituted C₁₋₂ alkyl group, or may be independently -R₁₀-(L₄)ₙ-R₁₁,
each R₁₀ may be independently a substituted or unsubstituted C₆₋₈ alkylene group,
each R₁₁ may be independently a substituted or unsubstituted, saturated or unsaturated monovalent C₅₋₁₅ hydrocarbon group,
each of L₁, L₂, L₃ and L₄ may be independently selected from the group consisting of - C(O)O-, -OC(O)-, -C(O)N(R')-, -N(R')C(O)-, -P(O)(OR')O-, -S-S-, and C₂₋₅ alkenylene, wherein each R' may be independently selected from the group consisting of hydrogen atom and C₁₋₆ alkyl,
n is 0 or 1, and
X⁻ may be Cl⁻, Br⁻, or acetate anion (CH₃COO⁻) (i.e., AcO⁻).

Even more specifically, the cationic lipid may be one having a structure selected from the following Formulas A to Q:

| Formula | Structure |
|---|---|
| A | 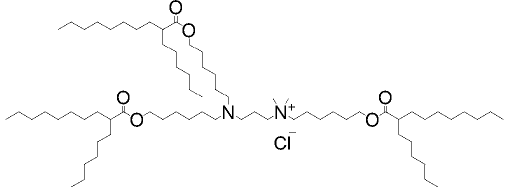 |
| B | 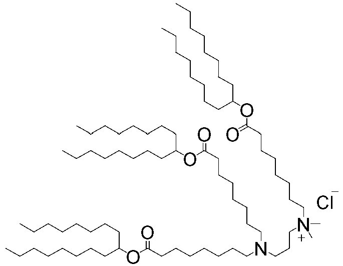 |
| C | 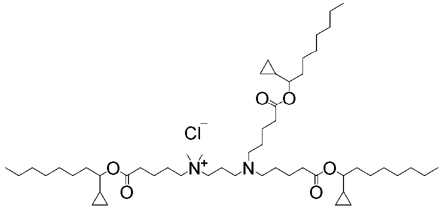 |
| D | 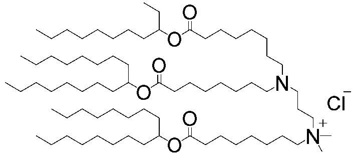 |
| E | 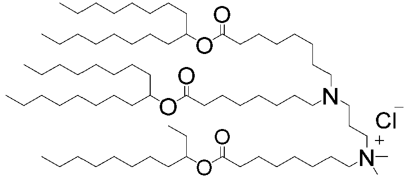 |
| F | 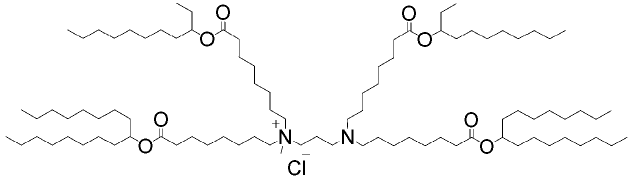 |
| G | 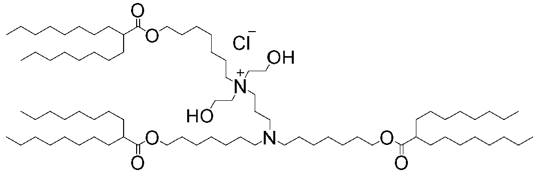 |
| H | 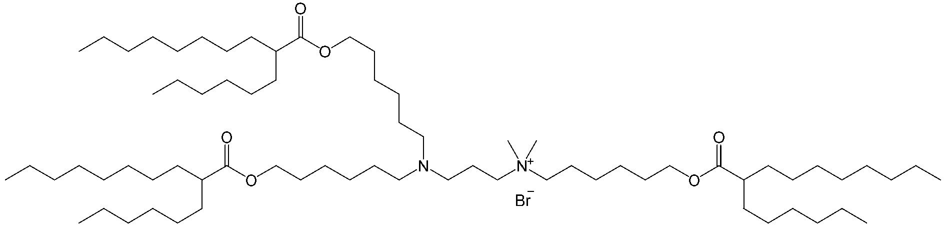 |
| I | 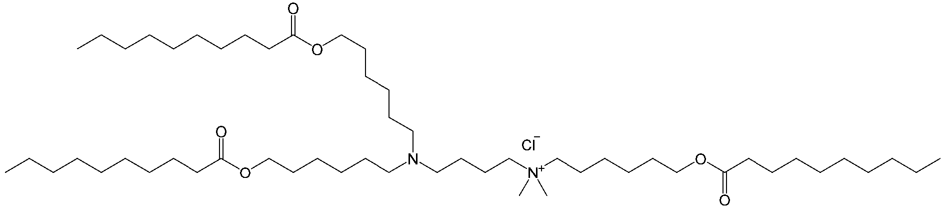 |
| J | 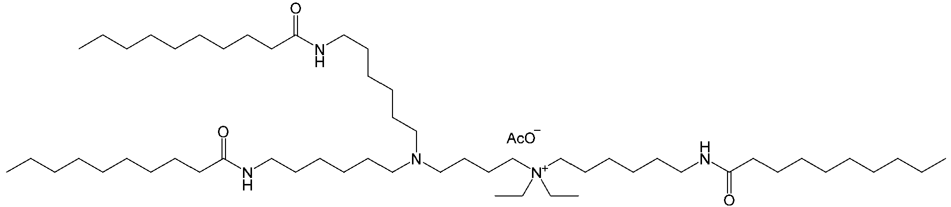 |
| K | 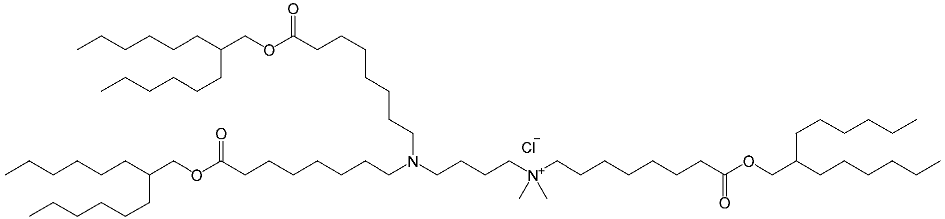 |
| L | 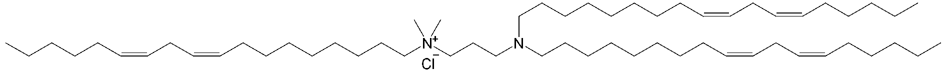 |
| M | 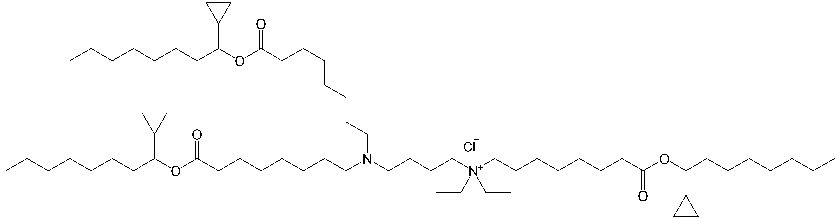 |
| N | 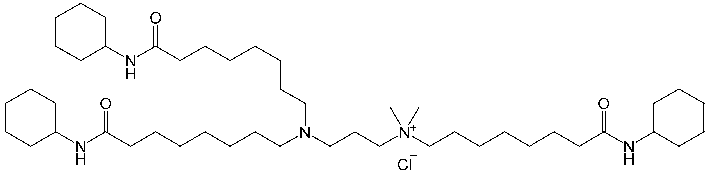 |
| O | 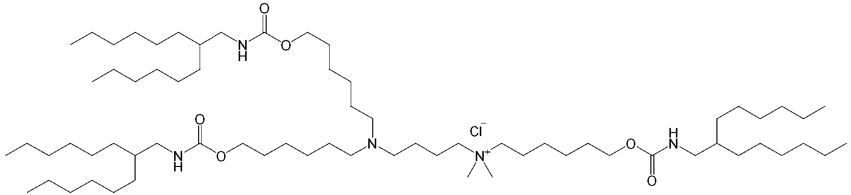 |
| P | 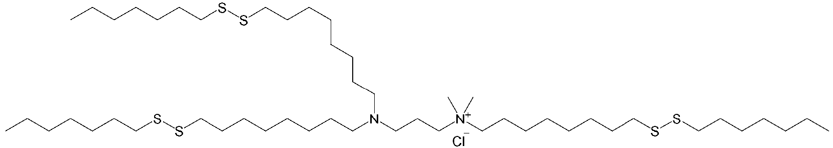 |
| Q | 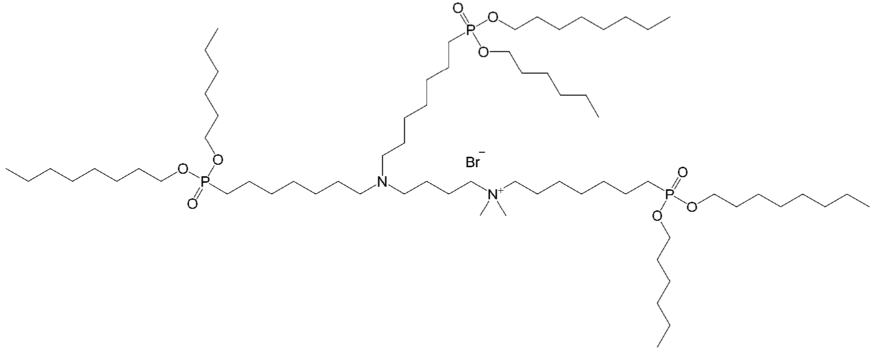 |

In an embodiment, the amount of the cationic lipid in the composition for drug delivery of the present invention may be, based on the dry weight of the total composition, 5 wt% or more, 10 wt% or more, 15 wt% or more, 20 wt% or more, 25 wt% or more, 30 wt% or more, or 35 wt% or more, and it may also be 95 wt% or less, 90 wt% or less, 85 wt% or less, 80 wt% or less, 75 wt% or less, 70 wt% or less, or 65 wt% or less. If the amount of the cationic lipid is too less, it may not be sufficient to form nanoparticles. To the contrary, if the amount of the cationic lipid is too much, the size of the nanoparticle becomes too large, and thus the nanoparticle stability may be lowered and the rate of loss during filter sterilization may increase.

The cationic lipid having the structure represented by Formula 1 may serve both as an ionizable lipid and as a helper lipid in the composition for drug delivery. The term "ionizable lipid" refers to a lipid capable of modulating its charge depending on pH and is one of the key components of lipid nanoparticle compositions useful for delivering nucleic acids such as RNA, DNA, etc. The term "helper lipid" refers to a lipid that enhances the particle stability and fluidity of lipid nanoparticles and may encompass, in addition to the ionizable lipid, various types of lipids such as phospholipids included in lipid nanoparticles.

However, even though the cationic lipid having the structure represented by Formula 1 may perform the functions of both an ionizable lipid and a helper lipid, the use of such additional lipid components is not excluded from the present invention. Accordingly, the composition for drug delivery of the present invention may further comprise, as needed, one or more ionizable lipids other than the cationic lipid having the structure represented by Formula 1 or one or more helper lipids other than the cationic lipid having the structure represented by Formula 1.

### Polymer

The composition for drug delivery of the present invention comprises lipid-polymer, amphiphilic block copolymer, or a combination thereof, as polymer component.

The lipid-polymer is a polymer having both hydrophilic and hydrophobic parts within the polymer molecule.

In an embodiment, the lipid-polymer may be a polymer in which one or more saturated or unsaturated hydrocarbon groups having 11 to 25 carbon atoms as a hydrophobic part are introduced into a hydrophilic block which is a hydrophilic part.

The hydrophilic block may be one or more selected from the group consisting of polyalkylene glycol, polyvinyl alcohol, polyvinyl pyrrolidone, polyacrylamide, and derivatives thereof.

More specifically, the hydrophilic block may be one or more selected from the group consisting of monomethoxypolyethylene glycol, monoacetoxypolyethylene glycol, polyethylene glycol, copolymer of polyethylene and propylene glycol, and polyvinylpyrrolidone.

In an embodiment, the number average molecular weight (g/mol) of the hydrophilic block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic block may be chemically combined with a functional group or ligand capable of reaching specific tissue or cell, or a functional group capable of promoting intracellular delivery, in order to control *in vivo* distribution of the nanoparticle carrier or to increase the efficiency of delivering the nanoparticle carrier into cell. The functional group or ligand may be one or more selected from the group consisting of monosaccharides, polysaccharides, vitamins, peptides, proteins, and antibodies to cell surface receptors. More specifically, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

In an embodiment, the saturated or unsaturated hydrocarbon group having 11 to 25 carbon atoms, the hydrophobic part introduced into a hydrophilic block which is the hydrophilic part, may independently be selected from the group consisting of myristoyl, dimyristoyl, lauryl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, and docosahexaenyl.

Also, in an embodiment, in the lipid-polymer, the amount ratio of the hydrophilic part and the hydrophobic part may be 10 to 90 wt%, more specifically 20 to 80 wt%, even more specifically 30 to 80 wt%, and even more specifically 40 to 80 wt% of the hydrophilic part, based on the weight of the polymer. If the ratio of the hydrophilic part is too small, the solubility of the polymer in water is low, making it difficult to form nanoparticles, and to the contrary, if it is too large, the hydrophilicity becomes too high, which may lower the stability of the nanoparticles.

In an embodiment of the present invention, the lipid-polymer may be a polyalkylene glycol (e.g., polyethylene glycol) into which saturated or unsaturated hydrocarbon group having 11 to 25 carbon atoms (e.g., myristyl group) has been introduced. For example, the lipid-polymer may be a PEG lipid (PEGylated lipid). The PEG lipid refers to a polyethylene glycol (PEG)-modified lipid, and is a type of PEG derivative having a lipid moiety attached thereto, such as DMG (dimyristoylglycerol) or DSPE (distearoyl glycerophosphoethanolamine). The PEG lipid may be used to improve the circulation time of active ingredient encapsulated in a lipid nanoparticle and to reduce non-specific uptake. The PEG lipid may be one or more selected from the group consisting of PEG-modified phosphatidylethanolamines, PEG-modified phosphatidic acids, PEG-modified ceramides, PEG-modified dialkylamines, PEG-modified diacylglycerols, and PEG-modified dialkylglycerols, and combinations thereof. More specifically, the PEG lipid may include 1,2-dimyristoyl-sn-glycero methoxypolyethylene glycol (PEG-DMG), 1,2-distearoyl-sn-glycero-3-phosphoethanolamine-N-[amino(polyethylene glycol)] (PEG-DSPE), PEG-distearyl glycerol (PEG-DSG), PEG-dipalmitoyl, PEG-dioleyl, PEG-distearyl, PEG-diacylglycamide (PEG-DAG), PEG-dipalmitoyl phosphatidylethanolamine (PEG-DPPE), or PEG-1,2-dimyristyloxypropyl-3-amine (PEG-c-DMA).

The amphiphilic block copolymer may be an A-B type block copolymer comprising a hydrophilic A block and a hydrophobic B block. In an aqueous environment, the A-B type block copolymer forms core-shell type polymer nanoparticle wherein the hydrophobic B block forms the core (inner wall) and the hydrophilic A block forms the shell (outer wall).

In an embodiment, the hydrophilic A block may be one or more selected from the group consisting of polyalkylene glycol, polyvinyl alcohol, polyvinyl pyrrolidone, polyacrylamide, and derivatives thereof.

More specifically, the hydrophilic A block may be one or more selected from the group consisting of monomethoxypolyethylene glycol (mPEG), monoacetoxypolyethylene glycol, polyethylene glycol, copolymer of polyethylene and propylene glycol, and polyvinylpyrrolidone.

In an embodiment, the number average molecular weight (g/mol) of the hydrophilic A block may be 200 or more, 500 or more, 1,000 or more, or 2,000 or more, and it also may be 50,000 or less, 20,000 or less, 10,000 or less, or 5,000 or less, but it is not limited thereto.

Also, if necessary, the end of the hydrophilic A block may be chemically combined with a functional group or ligand capable of reaching specific tissue or cell, or a functional group capable of promoting intracellular delivery, in order to control *in vivo* distribution of polymer nanoparticle carrier formed by the amphiphilic block copolymer and salt of polylactic acid or to increase the efficiency of delivering the nanoparticle carrier into cell. In an embodiment, the functional group or ligand may be one or more selected from the group consisting of monosaccharides, polysaccharides, vitamins, peptides, proteins, and antibodies to cell surface receptors. More specifically, the functional group or ligand may be one or more selected from the group consisting of anisamide, vitamin B9 (folic acid), vitamin B12, vitamin A, galactose, lactose, mannose, hyaluronic acid, RGD peptide, NGR peptide, transferrin, antibody to transferrin receptor, etc.

The hydrophobic B block is a biocompatible, biodegradable polymer, and in an embodiment, it may be one or more selected from the group consisting of polyester, polyanhydride, polyamino acid, polyorthoester and polyphosphazine.

More specifically, the hydrophobic B block may be one or more selected from the group consisting of polylactide (PLA), polyglycolide, polycaprolactone, polydioxan-2-one, copolymer of polylactide and polyglycolide, copolymer of polylactide and polydioxan-2-one, copolymer of polylactide and and polycaprolactone, and a copolymer of polyglycolide and polycaprolactone.

In an embodiment, the number average molecular weight (g/mol) of the hydrophobic B block may be 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic A block-hydrophobic B block may be 2,000-6,000, 2,000-4,000, 2,000-3,000, 2,000-1,700, 2,000-1,300, etc., but it is not limited thereto.

Also, in an embodiment, in order to increase the hydrophobicity of and thereby improve the stability of the nanoparticle, the hydrophobic B block may be modified by chemically combining the hydroxyl group at the end of the hydrophobic B block with tocopherol, cholesterol, or fatty acids having 10 to 24 carbons.

In an embodiment, in the amphiphilic block copolymer, the amount ratio of the hydrophilic block (A) and the hydrophobic block (B) may be such as 20 to 70 wt% and more specifically 30 to 60 wt% of the hydrophilic block (A), based on the weight of the copolymer. If the ratio of the hydrophilic block (A) is less than 20 wt%, the solubility of the polymer in water is low, making it difficult to form nanoparticles. Thus, in order for the copolymer to have sufficient water solubility to form nanoparticles, it is preferable that the ratio of the hydrophilic block (A) be 20 wt% or more. To the contrary, if the ratio of the hydrophilic block (A) is greater than 70 wt%, the hydrophilicity becomes too high, lowering the stability of the nanoparticles and making it difficult to use as a solubilizing composition of effective ingredient/lipid complex. Thus, considering the stability of the nanoparticles, it is preferable that the ratio of the hydrophilic block (A) be 70 wt% or less.

In an embodiment, the amphiphilic block copolymer may be a biocompatible, biodegradable polymer comprising the hydrophilic block described above; and a hydrophobic block having a repeating unit represented by the following Formula 2: 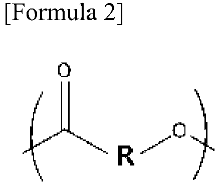 wherein, in the above Formula 2,
R represents a branched alkylene group having 3 or more carbon atoms.

In an embodiment, the number of repeating units (degree of polymerization) of the hydrophobic block having a repeating unit represented by the above Formula 2 may be, for example, 1 or more, 2 or more, 3 or more, 4 or more, 5 or more, 6 or more, 7 or more, 8 or more, 9 or more, or 10 or more, and may also be 25 or less, 24 or less, 23 or less, 22 or less, 21 or less, or 20 or less, but is not limited thereto.

In an embodiment, the number of carbon atoms of R in the above Formula 2 may be, for example, 3 or more, 4 or more, 5 or more, 6 or more, or 7 or more, and may also be 20 or less, 19 or less, 18 or less, 17 or less, 16 or less, 15 or less, 14 or less, or 13 or less, but is not limited thereto.

In an embodiment, R in the above Formula 2 may represent a branched alkylene group having 3 to 20 carbon atoms, more specifically a branched alkylene group having 3 to 17 carbon atoms, still more specifically a branched alkylene group having 3 to 15 carbon atoms, and even more specifically a branched alkylene group having 3 to 13 carbon atoms, but is not limited thereto.

In an embodiment, the hydrophobic block having a repeating unit represented by the above Formula 2 may be a biocompatible, biodegradable polymer having repeating units of a structure selected from the following, but is not limited thereto:

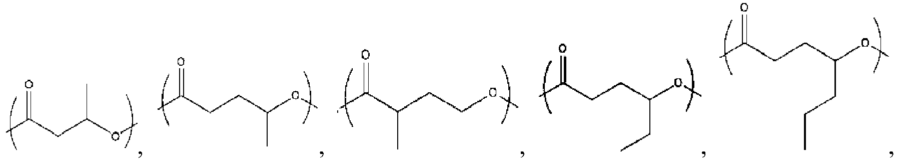

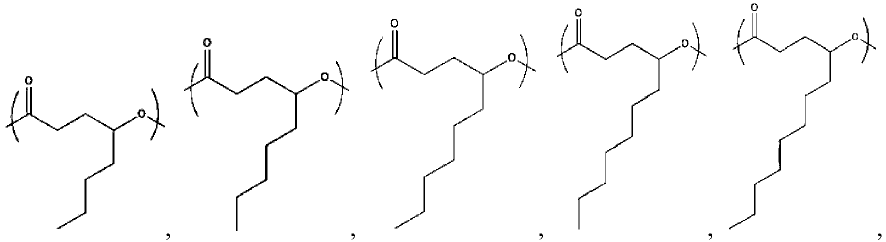

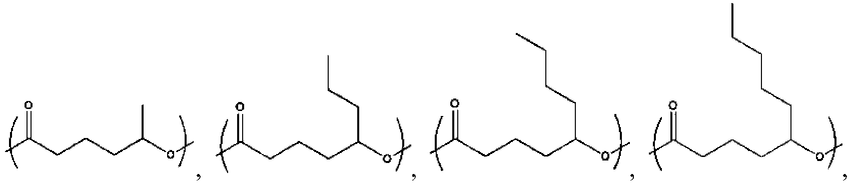

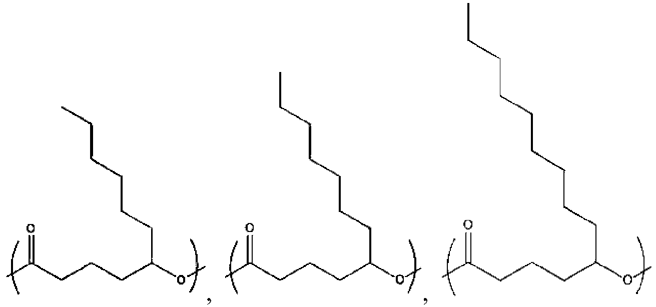

In an embodiment, the repeating unit represented by the above Formula 2 may be obtained by ring-opening polymerization of a lactone compound.

In an embodiment, the number average molecular weight (g/mol) of the hydrophobic block having a repeating unit represented by the above Formula 2 may be 80 or more, 100 or more, 150 or more, 200 or more, 500 or more, 1,000 or more, or 1,700 or more, and it also may be 50,000 or less, 20,000 or less, 10,000 or less, or 6,000 or less, but it is not limited thereto.

For example, the number average molecular weight combination of the hydrophilic block and the hydrophobic block having a repeating unit represented by the above Formula 2 may be 2,000-6,000, 2,000-4,000, 2,000-3,000, 2,000-1,700, 2,000-1,000, 2,000-800, 2,000-500, etc., but it is not limited thereto.

Also, in an embodiment, in order to increase the hydrophobicity of the hydrophobic block having a repeating unit represented by the above Formula 2 and thereby improve the stability of the nanoparticle, the hydrophobic block may be modified by chemically combining the hydroxyl group at the end of the hydrophobic block with tocopherol, cholesterol, or a fatty acid having 10 to 24 carbon atoms.

In an embodiment, in the amphiphilic block copolymer comprising the hydrophobic block having a repeating unit represented by the above Formula 2, the amount ratio of the hydrophilic block and the hydrophobic block may be such that the hydrophilic block is present in an amount of 25 to 95 wt%, specifically 40 to 90 wt%, and more specifically 50 to 80 wt%, based on the total weight of the copolymer. If the ratio of the hydrophilic block is less than 25 wt% based on the total weight of the copolymer, the solubility of the polymer in water is low, making it difficult to form nanoparticles. Thus, in order for the copolymer to have sufficient water solubility to form nanoparticles, it is preferable that the ratio of the hydrophilic block be 25 wt% or more. To the contrary, if the ratio of the hydrophilic block is greater than 95 wt% based on the total weight of the copolymer, the hydrophilicity becomes too high, lowering the stability of the polymer nanoparticles and making it difficult to use the copolymer as a solubilizing composition of effective ingredient-containing complex. Thus, considering the stability of the nanoparticles, it is preferable that the ratio of the hydrophilic block be 95 wt% or less.

In an embodiment, the amount of the polymer, which is lipid-polymer, amphiphilic block copolymer, or a combination thereof, in the composition for drug delivery of the present invention may be, based on the dry weight of the total composition, 5 wt% or more, 7 wt% or more, 10 wt% or more, 12 wt% or more, 15 wt% or more, 17 wt% or more, or 20 wt% or more, and it may also be 90 wt% or less, 80 wt% or less, 70 wt% or less, 60 wt% or less, 50 wt% or less, or 40 wt% or less. If the amount of the polymer is too less, the size of the nanoparticle becomes too large, and thus the nanoparticle stability may be lowered and the rate of loss during filter sterilization may increase. To the contrary, if the amount of the polymer is too much, there is a concern that the amount of the effective ingredient that can be incorporated will become too less.

In the composition for drug delivery of the present invention, the effective ingredient is maintained in a state of being encapsulated within a nanoparticle structure formed by the polymer, which is lipid-polymer, amphiphilic block copolymer, or a combination thereof, and the cationic lipid of the above Formula 1, thereby improving stability in blood or body fluids.

In an embodiment, the particle size of the nanoparticle can be defined by Z-average value, and for example, it may be 800 nm or less, 600 nm or less, 500 nm or less, 400 nm or less, 300 nm or less, 200 nm or less, or 150 nm or less, and also may be 10 nm or more, 20 nm or more, 30 nm or more, 40 nm or more, 50 nm or more, 60 nm or more, 70 nm or more, 80 nm or more, 90 nm or more, or 100 nm or more. In an embodiment, the particle size of the nanoparticle defined by Z-average value may be, for example, 10 to 800 nm, 20 to 600 nm, 30 to 500 nm, 40 to 400 nm, or 50 to 300 nm.

In an embodiment, the relative amount of the polymer, which is lipid-polymer, amphiphilic block copolymer, or a combination thereof, to the lipid of the above Formula 1 may be, based on 1 part by weight of the lipid of Formula 1, 0.01 part by weight or more, 0.02 part by weight or more, 0.03 part by weight or more, 0.04 part by weight or more, or 0.05 part by weight or more, and it may also be 50 parts by weight or less, 49 parts by weight or less, 47 parts by weight or less, 45 parts by weight or less, 43 parts by weight or less, 41 parts by weight or less, 40 parts by weight or less, 39 parts by weight or less, or 37 parts by weight or less, but it is not limited thereto.

More specifically, the relative amount of the lipid-polymer to the lipid of the above Formula 1 may be, based on 1 part by weight of the lipid of the above Formula 1, 0.01 part by weight or more, 0.02 part by weight or more, 0.03 part by weight or more, 0.04 part by weight or more, or 0.05 part by weight or more, and it may also be 5 parts by weight or less, 2 parts by weight or less, 1 part by weight or less, 0.5 part by weight or less, or 0.1 part by weight or less, but it is not limited thereto.

More specifically, the relative amount of the amphiphilic block copolymer to the lipid of the above Formula 1 may be, based on 1 part by weight of the lipid of the above Formula 1, 0.1 part by weight or more, 0.5 part by weight or more, 1 part by weight or more, 2 parts by weight or more, or 3 parts by weight or more, and it may also be 50 parts by weight or less, 40 parts by weight or less, 30 parts by weight or less, 20 parts by weight or less, or 10 parts by weight or less, but it is not limited thereto.

### Optional additive component

In an embodiment, in order to increase the efficiency of *in vivo* delivery of the effective ingredient, the composition for drug delivery of the present invention may further comprise fusogenic lipid.

In an embodiment, the fusogenic lipid may be one or a combination of two or more selected from the group consisting of phospholipid, cholesterol, and tocopherol.

Specifically, the phospholipid may be one or more selected from the group consisting of phosphatidylethanolamine (PE), phosphatidylcholine (PC) and phosphatidic acid. The phosphatidylethanolamine (PE), phosphatidylcholine (PC) and phosphatidic acid may be in a form combined with one or two C₁₀₋₂₄ fatty acids. The cholesterol and tocopherol include analogues, derivatives and metabolites of each of the cholesterol and tocopherol.

More specifically, the fusogenic lipid may be one or a combination of two or more selected from the group consisting of dilauroyl phosphatidylethanolamine, dimyristoyl phosphatidylethanolamine, dipalmitoyl phosphatidylethanolamine, distearoyl phosphatidylethanolamine, dioleoyl phosphatidylethanolamine, dilinoleoyl phosphatidylethanolamine, 1-palmitoyl-2-oleoyl phosphatidylethanolamine, 1,2-diphytanoyl-3-sn-phosphatidylethanolamine, dilauroyl phosphatidylcholine, dimyristoyl phosphatidylcholine, dipalmitoyl phosphatidylcholine, distearoyl phosphatidylcholine, dioleoyl phosphatidylcholine, dilinoleoyl phosphatidylcholine, 1-palmitoyl-2-oleoyl phosphatidylcholine, 1,2-diphytanoyl-3-sn-phosphatidylcholine, dilauroyl phosphatidic acid, dimyristoyl phosphatidic acid, dipalmitoyl phosphatidic acid, distearoyl phosphatidic acid, dioleoyl phosphatidic acid, dilinoleoyl phosphatidic acid, 1-palmitoyl-2-oleoyl phosphatidic acid, 1,2-diphytanoyl-3-sn-phosphatidic acid, cholesterol, and tocopherol.

Still more specifically, the fusogenic lipid may be one or a combination of two or more selected from the group consisting of dioleoyl phosphatidylethanolamine (DOPE), 1,2-dipalmitoleoyl-sn-glycero-3-phosphocholine (DPPC), distearoyl phosphatidylcholine (DSPC), 1,2-dioleoyl-sn-glycero-3-phosphocholine (DOPC), 1,2-dipalmitoleoyl-sn-glycero-3-phosphoethanolamine (DPPE), 1,2-dioleoyl-3-trimethylammonium-propane (DOTAP), 1,2-dioleoyl-3-dimethylammonium propane (DODAP), 1,2-dioleoyl-sn-glycero-3-phosphate (18PA), cholesterol, and tocopherol.

In an embodiment of the present invention, the fusogenic lipid may be distearoyl phosphatidylcholine, cholesterol, or a combination thereof.

In an embodiment, the amount of the fusogenic lipid may be, based on the dry weight of the total composition, 1 wt% or more, 2 wt% or more, 3 wt% or more, 4 wt% or more, or 5 wt% or more, and it may also be 40 wt% or less, 35 wt% or less, 30 wt% or less, 25 wt% or less, or 20 wt% or less.

In an embodiment, the relative amount of the fusogenic lipid to the cationic lipid of the above Formula 1 may be, based on 1 part by weight of the cationic lipid of Formula 1, 0.05 part by weight or more, 0.06 part by weight or more, 0.07 part by weight or more, 0.08 part by weight or more, 0.09 part by weight or more, or 0.1 part by weight or more, and it may also be 6 parts by weight or less, 5.5 parts by weight or less, 5 parts by weight or less, 4.5 parts by weight or less, 4 parts by weight or less, or 3.8 parts by weight or less, but it is not limited thereto.

In an embodiment, in case of using phospholipid as the fusogenic lipid, its relative amount may be, based on 1 part by weight of the lipid of Formula 1, 0.03 part by weight or more, 0.04 part by weight or more, 0.05 part by weight or more, or 0.06 part by weight or more, and it may also be 4 parts by weight or less, 3.9 parts by weight or less, 3.7 parts by weight or less, 3.5 parts by weight or less, 3.3 parts by weight or less, 3.1 parts by weight or less, 3 parts by weight or less, 2.9 parts by weight or less, or 2.7 parts by weight or less, but it is not limited thereto.

In an embodiment, in case of using cholesterol as the fusogenic lipid, its relative amount may be, based on 1 part by weight of the cationic lipid of Formula 1, 0.02 part by weight or more, 0.03 part by weight or more, or 0.04 part by weight or more, and it may also be 2 parts by weight or less, 1.9 parts by weight or less, 1.7 parts by weight or less, 1.5 parts by weight or less, 1.3 parts by weight or less, or 1.1 parts by weight or less, but it is not limited thereto.

Also, in an embodiment, the composition for drug delivery of the present invention may further comprise one or more additive components commonly included in a drug delivery composition (hereinafter, "optional additive component(s)").

In an embodiment, the optional additive component(s) may be, for example, one or more selected from the group consisting of pH modifiers (e.g., acidifying agents, alkalizing agents, buffering agents), tonicity modifiers, bulking agents (e.g., sugars, polyols, amino acids, polymers, proteins, etc.), wetting agents, solubilizing agents, surfactants, antioxidants, antimicrobial agents, chelating agents, complexing agents, etc., but it is not limited thereto.

In an embodiment, the pH modifier may be one or more selected from the group consisting of acetate, citrate, tartrate, histidine, glutamate, phosphate, Tris, glycine, bicarbonate, succinate, sulfate, nitrate, etc., but it is not limited thereto.

In an embodiment, the tonicity modifier may be one or more selected from the group consisting of mannitol, sorbitol, lactose, dextrose, trehalose, sodium chloride, potassium chloride, glycerol, glycerin, propylene glycol, etc., but it is not limited thereto.

In an embodiment, the bulking agent may be one or more selected from the group consisting of sugars and polyols including sucrose, trehalose, glucose, lactose, sorbitol, mannitol, glycerol, etc.; amino acids including arginine, aspartic acid, glutamic acid, lysine, proline, glycine, histidine, methionine, alanine, etc.; polymers and proteins including gelatin, polyvinylpyrrolidone (PVP), poly(lactide-co-glycolide) (PLGA), polyethylene glycol (PEG), dextran, cyclodextran, or derivatives thereof, starch derivatives, hydroxylamine sulfate (HAS), bovine serum albumin (BSA), etc.; or combinations thereof, but it is not limited thereto.

In an embodiment, the wetting agent and/or solubilizing agent may be one or more selected from the group consisting of lecithin, PEG 300, PEG 600, PEG 1000, polyoxyethylene lauryl ethers (e.g., Brij 30, Brij 35, Brij 56, Brij 76, Brij 97), polypropylene glycol (PPG) 2000, glucoside alkyl ethers, polyoxyethylene glycol octylphenol ethers, polyoxyethylene glycol alkylphenol ethers, glycerol alkyl esters, polysorbate 20, polysorbate 40, polysorbate 60, polysorbate 80, sorbitan monolaurate (Span 20), sorbitan monooleate (Span 80), sorbitan trioleate (Span 85), cocamide MEA, cocamide DEA, dodecyldimethylamine oxide, poloxamer, polyvinyl pyrrolidone K25, polyvinyl alcohol, oligolactic acid, sodium dioctyl sulfosuccinate, diethylene glycol monolaurate, triethanolamine oleate, sodium oleate, potassium oleate, ethyl oleate, oleic acid, ethyl laurate, sodium lauryl sulfate, etc., but it is not limited thereto.

In an embodiment, the antioxidant may be one or more selected from the group consisting of tocopherol (vitamin E), alpha tocopherol, alpha tocopherol hydrogen succinate, ascorbic acid, ascorbyl palmitate, butylated hydroxy anisole (BHA), butylated hydroxy toluene (BHT), monothioglycerol, potassium metabisulfite, propionic acid, propyl gallate, sodium ascorbate, sodium bisulfite, sodium metabisulfite, sodium sulfite, histamine, methionine, glutathione, poly(ethylamine), etc., but it is not limited thereto.

In an embodiment, the antimicrobial agent may be one or more selected from the group consisting of benzalkonium chloride, benzethonium chloride, benzyl alcohol, bronopol, cetrimide, cetylpyridinium chloride, chlorhexidine, chlorobutanol, chlorocresol, chloroxylenol, cresol, metacresol, ethyl alcohol, glycerin, hexetidine, imidurea, phenol, phenoxyethanol, phenylethyl alcohol, phenylmercuric nitrate, propylene glycol, thimerosal, etc., but it is not limited thereto.

In an embodiment, the chelating agent may be one or more selected from the group consisting of ethylenediaminetetraacetic acid (EDTA), edetate disodium, dipotassium edetate, edetic acid, fumaric acid, malic acid, phosphoric acid, sodium edetate, tartaric acid, diethylenetriamine pentaacetic acid (DPTA), citric acid, hexaphosphate, thioglycolic acid, zinc, etc., but it is not limited thereto.

When the optional additive component is used in the composition for drug delivery of the present invention, the amount of each additive may be, for example, 0.01 wt% or more, 0.05 wt% or more, or 0.1 wt% or more, and it may also be 10 wt% or less, 5 wt% or less, or 1 wt% or less, based on the dry weight of the total composition, but it is not limited thereto.

### Composition and preparation method thereof

The composition for drug delivery according to the present invention can be administered through routes of administration such as blood vessels, muscles, mucous membranes, subcutaneous, intradermal, oral, bone, transdermal, or local tissues, and can be formulated into various oral or parenteral formulations suitable for such routes of administration. Examples of the oral formulations include various ones such as tablets, capsules, powder formulations, liquid formulations, etc., and examples of the parenteral formulations include various ones such as eye drops, injections, etc., and in an embodiment, the composition may be an injection formulation. For example, when the composition according to the present invention is freeze-dried, it can be reconstituted with distilled water for injection, 0.9% physiological saline, 5% aqueous dextrose solution, etc. to produce an injection formulation.

The present invention also provides a method for preparing a composition for drug delivery, comprising the steps of: (a) preparing a solution in which a cationic lipid represented by the above Formula 1; and lipid-polymer, amphiphilic block copolymer, or a mixture thereof; are dissolved in a water-miscible organic solvent; and (b) to the solution prepared in step (a), adding effective ingredient selected from nucleic acid, polypeptide, virus, or combination thereof, and mixing them.

In an embodiment, the water-miscible organic solvent in step (a) may be ethanol.

In an embodiment, step (a) can be performed in a solution under acidic condition.

In an embodiment, step (b) may comprise: (b-1) a step of preparing a buffer solution containing the effective ingredient; and (b-2) a step of adding the buffer solution of the effective ingredient prepared in step (b-1) to the solution prepared in step (a), and mixing them.

In an embodiment, the mixing ratio of the buffer solution of the effective ingredient prepared in step (b-1) to the solution prepared in step (a) may be 1:1 to 1:5 in volume ratio, and more specifically, it may be 1:2 to 1:4.

In another embodiment, step (b) may comprise: (b-1) a step of adding the effective ingredient to the solution prepared in step (a); and (b-2) a step of adding a buffer solution to the resulting mixture of step (b-1) and mixing them.

In an embodiment, the method for preparing a composition for drug delivery may further comprise a step of adding a buffer for modifying pH, water for injection, or a combination thereof to the resulting mixture of step (b).

In another embodiment, the method for preparing a composition for drug delivery may further comprise a step of removing the solvent from the resulting mixture of step (b) and then adding a freeze-drying aid thereto and freeze-drying the resulting mixture.

The freeze-drying aid is added to help the freeze-dried composition maintain a cake shape or to help the composition dissolve uniformly within a short period of time during the reconstitution process after freeze-drying, and specifically, it may be one or more selected from the group consisting of sugars, amino acids, polymers and proteins; for example, it may be one or more selected from the group consisting of lactose, mannitol, sorbitol, and sucrose. The amount of the freeze-drying aid may be 1 to 90 wt%, more specifically 10 to 60 wt%, based on the total dry weight of the freeze-dried composition.

The present invention will be explained below in more detail with reference to the following Examples. However, the Examples are only to illustrate the invention, and the scope of the present invention is not limited thereby in any manner.

### [EXAMPLES]

### Preparation Example 1

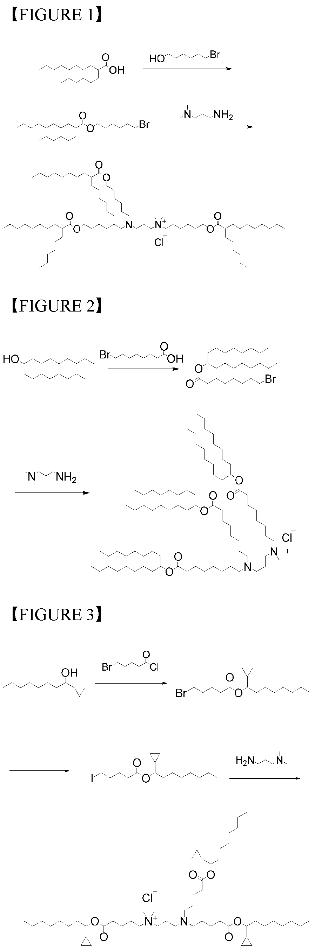

The compound of the following Formula A was prepared according to the synthesis scheme shown in Figure 1.

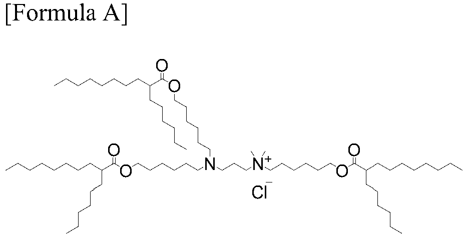

### (1) Synthesis of 6-bromohexyl 2-hexyldecanoate

In a 2000 mL 3-neck round-bottom flask (RBF), 2-hexyldecanoic acid (100 g, 390 mmol, 1.00 eq), 6-bromohexan-1-ol (91.8 g, 507 mmol, 1.30 eq) and toluene (1000 mL) were placed, and H₂SO₄ (7.65 g, 78.0 mmol, 0.20 eq) was added to the mixture. The mixture was degassed and purged with N₂ gas, and then stirred at 120°C for 16 hours. After cooling to room temperature, the mixture was concentrated under reduced pressure to remove the solvent. The residue was purified by silica column chromatography (petroleum ether:ethyl acetate (EtOAc)=10:1→1:100) to obtain 6-bromohexyl 2-hexyl decanoate (135 g, 322 mmol, 82.5% yield) in a form of pale yellow oil.

### (2) Synthesis of the compound of Formula A

In a 500 mL 3-neck RBF, 6-bromohexyl 2-hexyl decanoate (15.0 g, 35.8 mmol, 3.50 eq), N¹,N¹-dimethylpropane-1,3-diamine and ethanol (EtOH) (150 mL) were placed, and Na₂CO₃ (3.25 g, 30.7 mmol, 3.00 eq) was added to the mixture. After purging three times with N₂ gas, the mixture was stirred at 90°C for 48 hours. After cooling to room temperature, the solvent was removed under reduced pressure. The residue was extracted with a saturated aqueous solution of Na₂CO₃ and dichloromethane (DCM), and after taking the DCM layer, the solvent was removed under reduced pressure to obtain residue A. Residue A was dissolved in EtOH (60 mL), and H₂O (60 mL) and an excess amount of Na₂CO₃ were added thereto, and the mixture was stirred at room temperature for 16 hours. After filtering the mixture, the EtOH was removed from the filtrate under reduced pressure. The residual solution was extracted with DCM, and the DCM layer was taken, and the solvent was removed under reduced pressure to obtain residue B. Residue B was purified by reversed-phase HPLC (column: C1 250×80 mm, 10 µm; mobile phase: [HCl aqueous solution-ACN]; gradient: 55%-85% for 20 minutes) to obtain the compound of Formula A (12.0 g, 10.7 mmol, 35.5% yield) in a form of yellow solid.

¹H NMR (400 MHz, CHLOROFORM-*d*): *δ* 4.07-4.04 (m, 4H), 3.45 (td, 2H), 3.28 (s, 6H), 3.08 (br, 4H), 2.72 (br, H), 2.33-2.27 (m, 3H), 1.84 (br, 6H), 1.68-1.45 (m, 20H), 1.45-1.42 (m, 18H), 1.32-1.25 (m, 62H), 0.89 (t, 18H)

### Preparation Example 2

The compound of the following Formula B was prepared according to the synthesis scheme shown in Figure 2.

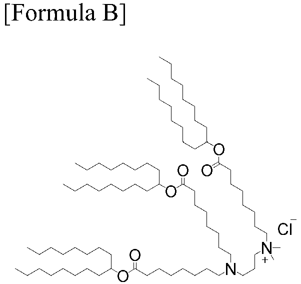

### (1) Synthesis of heptadecan-9-yl 8-bromooctanoate

In a 1000 mL of 3-neck RBF, heptadecan-9-ol (43.00 g, 168.00 mmol, 1.00 eq), 8-bromooctanic acid (44.90 g, 201.00 mmol, 1.20 eq), 4-dimethylaminopyridine (DMAP) (20.50 g, 168.00 mmol, 1.00 eq), 1-ethyl-3-(3-dimethylaminopropyl)carbodiimide (EDCI) (48.20 g, 252.00 mmol, 1.50 eq) and triethylamine (TEA) (17.00 g, 168.00 mmol, 23.30 mL, 1.00 eq) were placed, and DCM (430 mL) was added thereto. After purging with N₂ three times, the mixture was stirred under N₂ at 25°C for 16 hours. 500 mL of water was added to the reaction mixture at 20°C, and after quenching, the mixture was extracted with 900 mL of DCM (300 mL * 3). The collected organic layer was concentrated under reduced pressure, and the obtained residue was then purified by silica column chromatography (petroleum ether:EtOAc = 100:1→1:1) to obtain heptadecan-9-yl 8-bromooctanoate (36.00 g, 78.00 mmol, 46.5% yield) in a form of pale yellow oil.

### (2) Synthesis of the compound of Formula B

In a 100 mL of 3-neck RBF, heptadecan-9-yl 8-bromooctanoate (3.16 g, 6.85 mmol, 3.50 eq), N¹,N¹-dimethylpropane-1,3-diamine (0.20 g, 1.96 mmol, 1.00 eq), and N,N-diisopropylethylamine (DIEA) (1.26 g, 9.79 mmol, 1.70 mL, 5.00 eq) were placed, and EtOH (5 mL) was added thereto. After purging with N₂ three times, the mixture was stirred for 16 hours under N₂ condition at 95°C. The reaction mixture was filtered and concentrated under reduced pressure to remove the solvent. The mixture was extracted with saturated Na₂CO₃ and 300 mL (100 mL x 3) of DCM, and the collected organic layer was concentrated under reduced pressure to obtain residue A. Residue A was dissolved in EtOH (50 mL), then 60 mL of water and an excess amount of Na₂CO₃ were added to the mixture, and the mixture was stirred at 25°C for 16 hours. The mixture was filtered and concentrated under reduced pressure to remove EtOH, and then the aqueous layer was extracted with 300 mL (100 mL x 3) of DCM. The collected organic layer was concentrated under reduced pressure to obtain residue B. Residue B was purified by prep-HPLC (column: C1 250×80 mm, 10 µm; mobile phase: [HCl aqueous solution-ACN]; gradient: 55%-85% for 20 minutes) and concentrated under reduced pressure to remove the solvent, thereby to obtain the compound of Formula B (1.52 g, 1.19 mmol, 60.6% yield) in a form of pale yellow syrup.

¹H NMR (400 MHz, CHLOROFORM-*d*): *δ* 4.86 (t, *J*=6.2 Hz, 3H), 4.15 (br d, *J*=1.3 Hz, 2H), 3.44 - 3.32 (m, 4H), 3.28 (s, 6H), 3.14 - 2.94 (m, 4H), 2.72 - 2.58 (m, 2H), 2.28 (dt, *J*=2.1, 7.4 Hz, 6H), 1.96 - 1.74 (m, 12H), 1.65 - 1.58 (m, 6H), 1.51 (br d, *J*=5.8 Hz, 12H), 1.41 - 1.25 (m, 84H), 0.94 - 0.83 (m, 18H)

### Preparation Example 3

The compound of the following Formula C was prepared according to the synthesis scheme shown in Figure 3.

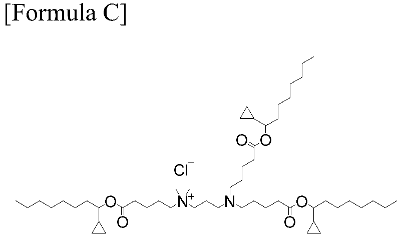

### (1) Synthesis of 1-cyclopropyloctyl 5-bromopentanoate

In a 500 mL of 3-neck RBF, 1-cyclopropyloctan-1-ol (15.00 g, 88.90 mmol, 1.00 eq), DMAP (2.15 g, 17.60 mmol, 0.20 eq) and TEA (17.80 g, 176.00 mmol, 24.50 mL, 2.00 eq) were placed, and DCM (150 mL) was added thereto. After purging the mixture with N₂ three times, 5-bromopentanoyl chloride (26.40 g, 132.00 mmol, 17.70 mL, 1.50 eq) was added to the solution at 0°C. After stirring the solution at 25°C for 16 hours, 100 mL of H₂O was added to the reaction mixture at 25°C for quenching. The mixture was extracted with 1500 mL of DCM (500 mL * 3), dried with Na₂SO₄, filtered, and concentrated under reduced pressure to obtain the residue. The residue was purified by silica column chromatography (petroleum ether:EtOAc=10:1) to obtain 1-cyclopropyloctyl 5-bromopentanoate (20.0 g, 60.0 mmol, 68.1% yield) in a form of colorless oil.

### (2) Synthesis of 1-cyclopropyloctyl 5-iodopentanoate

In a 500 mL 3-neck RBF, 1-cyclopropyloctyl 5-bromopentanoate (20.00 g, 60.00 mmol, 1.00 eq) and KI (19.90 g, 120.00 mmol, 2.00 eq) were placed, and acetonitrile (ACN) (200 mL) was added thereto. The mixture was purged with N₂ three times and stirred for 16 hours under N₂ condition at 90°C. The mixture was filtered and concentrated under reduced pressure to obtain 1-cyclopropyloctyl 5-iodopentanoate (18.00 g, 47.30 mmol, 78.9% yield) in a form of yellow oil.

### (3) Synthesis of the compound of Formula C

In a a 50 mL 3-neck RBF, N¹,N¹-dimethylpropane-1,3-diamine (0.20 g, 1.96 mmol, 0.33 eq), 1-cyclopropyloctyl 5-iodopentanoate (2.23 g, 5.87 mmol, 1.00 eq) and K₂CO₃ (812.00 mg, 5.87 mmol, 3.00 eq) were placed, and EtOH (2 mL) and ACN (2 mL) were added thereto. The mixture was purged with N₂ three times and stirred for 12 hours under N₂ condition at 80°C. The mixture was filtered and concentrated under reduced pressure to obtain the residue. The residue was purified by prep-HPLC (column: C1 250×80 mm, 10 µm; mobile phase: [HCl aqueous solution-ACN]; gradient: 55%-85% for 20 minutes) to obtain the compound of Formula C (0.19 g, 221.00 µmol, 11.3% yield) in a form of yellow syrup.

¹H NMR (400 MHz, CHLOROFORM-*d*): *δ* 0.26 (dt, *J*=9.57, 4.85 Hz, 3 H), 0.34 (dq, *J*=9.54, 4.87 Hz, 3 H), 0.40 - 0.49 (m, 3 H), 0.51 - 0.60 (m, 3 H), 0.83 - 0.92 (m, 9 H), 0.92 - 1.01 (m, 3 H), 1.27 (br s, 30 H), 1.39 - 1.47 (m, 4 H), 1.55 - 1.69 (m, 10 H), 1.70 - 1.78 (m, 2 H), 1.78 - 1.92 (m, 4 H), 2.32 (t, *J*=7.00 Hz, 4 H), 2.36 - 2.46 (m, 6 H), 2.50 (br t, *J*=5.88 Hz, 2 H), 3.38 (s, 6 H), 3.48 - 3.60 (m, 2 H), 3.60 - 3.74 (m, 2 H), 4.14 - 4.33 (m, 3 H)

### Preparation Example 4

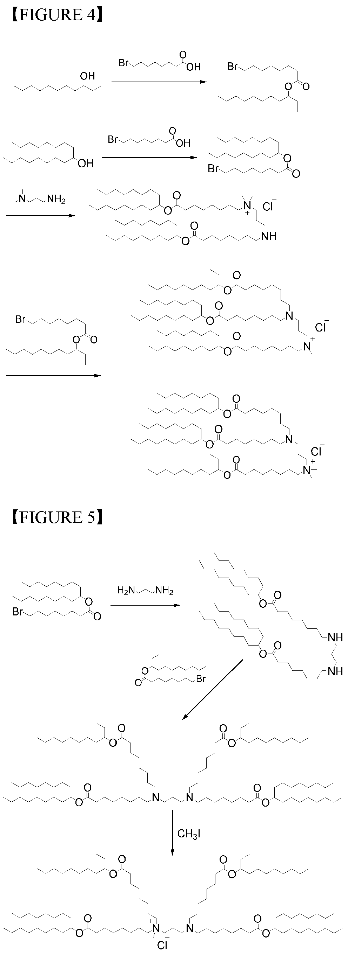

The compounds of the following Formulas D and E were prepared according to the synthesis scheme shown in Figure 4.

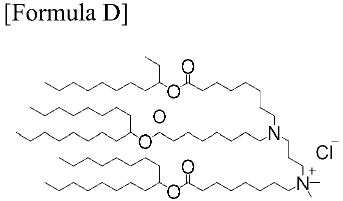

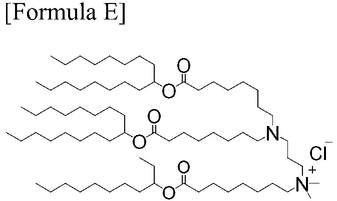

### (1) Synthesis of undecan-3-yl 8-bromooctanoate

In a 500 mL 3-neck RBF, undecane-3-ol (25.00 g, 145.00 mmol, 1.00 eq) dissolved in toluene (250 mL) was placed, and 8-bromooctanic acid (48.60 g, 218.00 mmol, 1.50 eq) was added thereto. Then, H₂SO₄ (2.13 g, 21.8 mmol, 0.15 eq) was added. The mixture was purged with N₂ three times and stirred at 120°C for 16 hours. The mixture was concentrated under reduced pressure to obtain the residue. The residue was purified by silica column chromatography (petroleum ether:EtOAc=10:1→1:100) to obtain undecane-3-yl 8-bromooctanoate (40.00 g, 106.00 mmol, 73.1% yield) in a form of yellow oil.

### (2) Synthesis of heptadecan-9-yl 8-bromooctanoate

In a 5000 mL 3-neck RBF, heptadecan-9-ol (235.00 g, 916.00 mmol, 1.00 eq) dissolved in toluene (2.35 L) was placed, and 8-bromooctanic acid (245.00 g, 1.10 mol, 1.20 eq) was added thereto. Then, H₂SO₄ (18.00 g, 183.00 mmol, 0.20 eq) was added. The mixture was purged with N₂ three times and stirred at 120°C for 16 hours. The mixture was concentrated under reduced pressure to obtain the residue. The residue was purified by silica column chromatography (petroleum ether:EtOAc=10:1→1:100) to obtain heptadecan-9-yl 8-bromooctanoate (470.00 g, 1.02 mol, 55.6% yield) in a form of yellow oil.

### (3) Synthesis of 8-(heptadecan-9-yloxy)-N-(3-((8-(heptadecan-9-yloxy)-8-oxooctyl)amino)propyl)-N,N-dimethyl-8-oxooctan-1-aminium chloride

In a 250 mL 3-neck RBF, heptadecan-9-yl 8-bromooctanoate (10.00 g, 21.70 mmol, 2.20 eq), N¹,N¹-dimethylpropane-1,3-diamine (1.01 g, 9.85 mmol, 1.00 eq), Na₂CO₃ (2.09 g, 19.70 mmol, 2.00 eq) and DIEA (3.82 g, 29.50 mmol, 3.00 eq) dissolved in EtOH (100 mL) were placed. After stirring the mixture at 90°C for 16 hours, the reaction mixture was concentrated under reduced pressure. The residue was purified by silica column chromatography (DCM:MeOH=10:1→1:100), and 6 g of the purified product was further purified by reverse-phase HPLC to obtain 8-(heptadecan-9-yloxy)-N-(3-((8-(heptadecan-9-yloxy)-8-oxo-octyl)amino)propyl)-N,N-dimethyl-8-oxo-octane-1-amine chloride (2.00 g, 2.31 mmol, 23.5% yield) in a form of yellow solid.

### (4) Synthesis of the compounds of Formulas D and E

In a 50 mL 3-neck RBF, undecane-3-yl 8-bromooctanoate (314.00 mg, 833.00 µmol, 1.20 eq), 8-(heptadecan-9-yloxy)-N-(3-((8-(heptadecan-9-yloxy)-8-oxo-octyl)amino)propyl)-N,N-dimethyl-8-oxo-octane-1-aminium chloride (0.60 g, 694.00 µmol, 1.00 eq) and K₂CO₃ (115.00 mg, 833.00 µmol, 1.20 eq) dissolved in ACN (6 mL) were placed. The mixture was stirred at 90°C for 24 hours, and then the reaction mixture was concentrated under reduced pressure. The residue was purified by purification using reverse-phase HPLC (column: C1 250×80 mm, 10 µm; mobile phase: [HCl aqueous solution-ACN]; gradient: 45%-75% for 25 minutes) to obtain the compound of Formula D (0.20 g, 167.00 µmol, 24.1% yield) in a form of yellow oil and the compound of Formula E (0.30 g, 258.00 µmol, 37.2% yield) in a form of white solid.

### [Compound of Formula D]

¹H NMR (400 MHz, CHLOROFORM-*d*): *δ* 11.60 (br s, 1H), 4.92 - 4.75 (m, 3H), 4.16 (br s, 2H), 3.48 - 3.33 (m, 4H), 3.29 (s, 6H), 3.14 - 2.96 (m, 4H), 2.66 (br s, 2H), 2.28 (tt, *J*=2.3, 7.3 Hz, 6H), 2.21 (br s, 2H), 1.82 (br s, 6H), 1.65 - 1.58 (m, 6H), 1.57 - 1.46 (m, 12H), 1.42 - 1.34 (m, 16H), 1.26 (br s, 54H), 0.88 (t, *J*=6.6 Hz, 18H)

### [Compound of Formula E]

¹H NMR (400 MHz, CHLOROFORM-*d*): *δ* 11.60 (br s, 1H), 4.90 - 4.76 (m, 3H), 4.14 (br d, *J*=7.1 Hz, 2H), 3.48 - 3.32 (m, 4H), 3.29 (s, 6H), 3.14 - 2.97 (m, 4H), 2.65 (br s, 2H), 2.32 - 2.25 (m, 8H), 1.82 (br s, 6H), 1.65 - 1.58 (m, 6H), 1.57 - 1.46 (m, 12H), 1.43 - 1.34 (m, 16H), 1.32 - 1.24 (m, 59H), 0.93 - 0.82 (m, 18H)

### Preparation Example 5

The compound of the following Formula F was prepared according to the synthesis scheme shown in Figure 5.

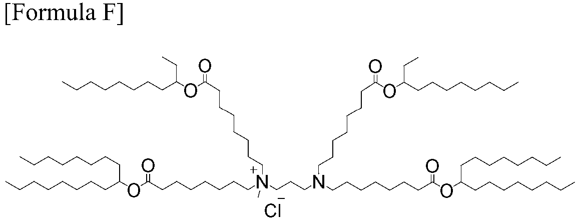

### (1) Synthesis of di(heptadecan-9-yl) 8,8'-(propane-1,3-diylbis(azanediyl))dioctanoate

In a 100 mL 3-neck RBF, heptadecan-9-yl 8-bromooctanoate (2.00 g, 4.33 mmol, 2.00 eq), propane-1,3-diamine (160.00 mg, 2.17 mmol, 1.00 eq) and K₂CO₃ (598.00 mg, 4.33 mmol, 2.00 eq) were placed, and ACN (20 mL) was added thereto. After purging the mixture with N₂ three times, the solution was stirred at 45°C for 16 hours. The reaction mixture was filtered and concentrated under reduced pressure to obtain the residue. The residue was purified by silica column chromatography (DCM:MeOH=10:1) to obtain di(heptadecan-9-yl) 8,8'-(propane-1,3-diylbis(azandyl))dioctanoate (0.80 g, 0.96 mmol, 11.0% yield) in a form of white solid.

### (2) Synthesis of di(heptadecan-9-yl) 8,8'-(9,33-diethyl-11,31-dioxo-10,32-dioxa-19,23-diazahentetracontane-19,23-diyl)dioctanoate

In a 50 mL 3-neck RBF, di(heptadecan-9-yl) 8,8'-(propane-1,3-diylbis(azandyl))dioctanoate (0.80 g, 0.96 mmol, 1.00 eq), undecane-3-yl 8-bromooctanoate (722.00 mg, 1.92 mmol, 1.00 eq) and K₂CO₃ (264.00 mg, 1.92 mmol, 2.00 eq) were placed, and ACN (8 mL) was added thereto. The solution was stirred at 55°C for 16 hours. The reaction mixture was filtered and concentrated under reduced pressure to obtain the residue. The residue was purified by prep-HPLC (water(HCl)-ACN) and concentrated under reduced pressure to remove ACN. An aqueous solution of NaHCO₃ (10 mL) was added to the aqueous solution from which ACN had been removed, and the mixture was neutralized overnight. Thereafter, the mixture was extracted with DCM (5 mL * 2), dried with Na₂SO₄, filtered, and subjected to reduced pressure to obtain di(heptadecan-9-yl) 8,8'-(9,33-diethyl-11,31-dioxo-10,32-dioxa-19,23-diazahentetracontan-19,23-diyl)dioctanoate (0.40 g, 0.28 mmol, 29.2% yield) in a form of yellow oil.

### (3) Synthesis of the compound of Formula F

In a 50 mL 3-neck RBF, di(heptadecan-9-yl) 8,8'-(9,33-diethyl-11,31-dioxo-10,32-dioxa-19,23-diazahentetracontan-19,23-diyl)dioctanoate (0.40 g, 0.28 mmol, 1.00 eq) dissolved in THF (4 mL) and K₂CO₃ (42.50 mg, 0.31 mmol, 1.10 eq) were placed, and CH₃I (39.70 mg, 0.28 mmol, 1.00 eq) was added thereto. The solution was stirred at 25°C for 16 hours. 2M HCl (10 mL) was added to the reaction mixture for quenching, and distilled water (DW) (5 mL) was added for dilution, followed by extraction with EtOAc (5 mL * 2). The collected organic layer was concentrated under reduced pressure to obtain the residue. The residue was purified by prep-HPLC (Column: C1 250×80 mm, 10 µm; Mobile phase: [HCl aqueous solution-ACN]; Gradient: 45%-75% for 25 minutes), and concentrated under reduced pressure to remove ACN. NaHCO₃ aqueous solution (10 mL) was added to the aqueous solution from which ACN had been removed, and the mixture was neutralized overnight. Subsequently, the mixture was extracted with DCM (5 mL * 2), dried with Na₂SO₄, filtered, and subjected to reduced pressure to obtain the compound of Formula F (0.40 g, 0.28 mmol, 29.2% yield) in a form of yellow oil.

¹HNMR: (400 MHz, CHLOROFORM-*d*) *δ* 0.74 - 1.00 (m, 24 H) 1.18 - 1.44 (m, 98 H) 1.47 - 1.56 (m, 14 H) 1.57 (br s, 8 H) 1.64 - 1.88 (m, 10 H) 2.18 - 2.38 (m, 9 H) 2.65 (ddd, *J*=10.98, 5.16, 1.88 Hz, 1 H) 2.90 - 3.15 (m, 3 H) 3.20 - 3.48 (m, 8 H) 3.92 - 4.12 (m, 2 H) 4.84 (dt, *J*=19.35, 6.14 Hz, 4 H)

### Preparation Example 6

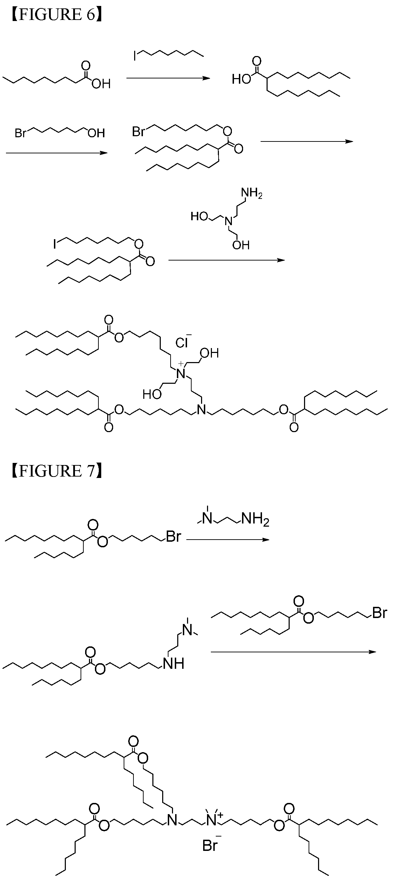

The compound of the following Formula G was prepared according to the synthesis scheme shown in Figure 6.

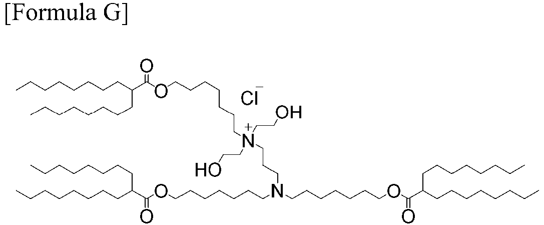

### (1) Synthesis of 2-octyldecanoic acid

In a 1000 mL 3-neck RBF, nonanoic acid (60.00 g, 348.00 mmol, 1.00 eq) and THF (100 mL) were placed under N₂, 0°C conditions. Lithium diisopropylamide (LDA) (2 M, 383.00 mL, 2.20 eq) was added to the mixture under N₂, 0°C conditions and the mixture was stirred at 0°C for 30 minutes. Subsequently, 1-iodooctane (92.00 g, 383.00 mmol, 1.00 eq) was added at room temperature and stirred at 45°C for 16 hours. The reaction mixture was quenched by adding 1 N HCl (1 L), extracted with EtOAc (2 L), dried with Na₂SO₄, filtered, and concentrated to obtain the residue. After purging with N₂ three times, the solution was stirred at 45°C for 16 hours. The residue was purified by silica column chromatography (petroleum ether:EtOAc=100:1→10:1) to obtain 2-octyldecanoic acid (67.00 g, 236.00 mmol, 67.6% yield) in a form of yellow oil.

### (2) Synthesis of 7-bromoheptyl 2-octyldecanoate

In a 250 mL 3-neck RBF, 7-bromoheptan-1-ol (5.14 g, 26.40 mmol, 1.50 eq) dissolved in toluene (50 mL), octyldecanoic acid (5.00 g, 17.60 mmol, 1.00 eq) and H₂SO₄ (259 mg, 2.64 mmol, 0.15 eq) were placed, and the mixture was purged with N₂ three times. The mixture was stirred at 120°C for 16 hours and concentrated under reduced pressure to obtain the residue. The residue was purified by silica column chromatography (petroleum ether:EtOAc=10:1→1:100) to obtain 7-bromoheptyl 2-octyldecanoate (5.50 g, 11.90 mmol, 67.8% yield) in a form of yellow oil.

### (3) Synthesis of 7-iodoheptyl 2-octyldecanoate

In a 250 mL 3-neck RBF, 7-bromoheptyl 2-octyl decanoate (5.50 g, 11.90 mmol, 1.00 eq) was placed, and ACN (55 mL) was added thereto. Then, KI (3.96 g, 23.80 mmol, 2.00 eq) was added, and the mixture was purged with N₂ three times. The mixture was stirred at 90°C for 24 hours, filtered, and concentrated under reduced pressure to obtain 7-iodoheptyl 2-octyl decanoate (5.00 g, 9.83 mmol, 82.5% yield) in a form of yellow oil.

### (4) Synthesis of the compound of Formula G

In a 250 mL 3-neck RBF, 7-iodoheptyl 2-octyldecanoate (5.00 g, 9.83 mmol, 4.00 eq) dissolved in ACN (50 mL), 2,2'-((3-aminopropyl)azandyl)bis(ethanol-1-ol) (399.00 mg, 2.46 mmol, 1.00 eq), and K₂CO₃ (42.50 mg, 0.31 mmol, 1.10 eq) were placed, and the mixture was purged with N₂ three times. The solution was stirred at 80°C for 16 hours, filtered, and concentrated under reduced pressure to obtain the residue. The residue was purified by prep-HPLC (column: C1 250×80 mm, 10 µm; mobile phase: [HCl aqueous solution-ACN]; gradient: 60%-90% for 25 minutes) to obtain the compound of Formula G (0.25 g, 0.19 mmol, 99.2% yield) in a form of gray oil.

¹H NMR (400 MHz, CHLOROFORM-*d*): *δ* 5.04 - 4.62 (m, 2H), 4.06 (t, *J*=6.8 Hz, 6H), 3.95 - 3.86 (m, 2H), 3.70 - 3.61 (m, 4H), 3.35 - 3.21 (m, 6H), 2.70 (br s, 2H), 2.61 (br s, 4H), 2.31 (tt, *J*=5.4, 8.8 Hz, 3H), 1.85 - 1.69 (m, 10H), 1.66 - 1.53 (m, 12H), 1.46 - 1.36 (m, 24H), 1.26 (s, 65H), 0.93 - 0.84 (m, 18H)

### Preparation Example 7

The compound of the following Formula H was prepared according to the synthesis scheme shown in Figure 7.

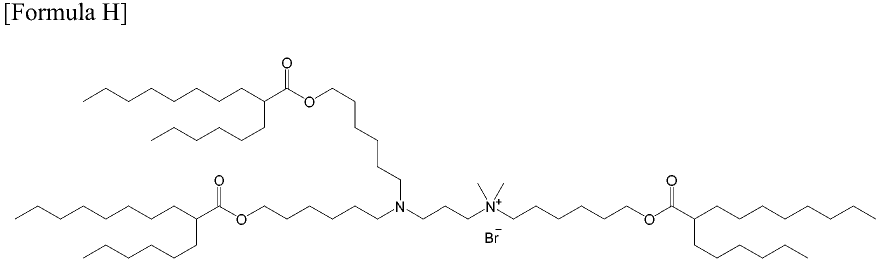

### (1) Synthesis of 6-((3-(dimethylamino)propyl)amino)hexyl 2-hexyldecanoate)

In a 100 mL 3-neck round-bottom flask (RBF), N¹,N¹-dimethylpropane-1,3-diamine (1.0 g, 9.79 mmol, 1.22 mL, 1 eq), 6-bromohexyl 2-hexyl decanoate (4.11 g, 9.79 mmol, 1 eq), and ethanol (20 mL) were placed. N,N-diisopropylethylamine (6.32 g, 48.93 mmol, 8.52 mL, 5.0 eq) was added to this mixture. The mixture was stirred at 90 °C for 3 days. After cooling to room temperature, silica (1 g) was added to the mixture, and the solvent was removed under vacuum. The residue was purified using a silica column with petroleum ether:EtOAc=1:1→2:1 to obtain 6-((3-(dimethylamino)propyl)amino)hexyl 2-hexyl decanoate) (1.08 g, 25.0% yield), in a form of pale yellow oil.

### (2) Synthesis of the compound of Formula H

In a 50 mL 3-neck RBF, 6-((3-(dimethylamino)propyl)amino)hexyl 2-hexyl decanoate) (1.08 g, 2.45 mmol, 1 eq), 6-bromohexyl 2-hexyl decanoate (1.03 g, 2.45 mmol, 1 eq) and ethanol (10 mL) were placed. N,N-diisopropylethylamine (633.40 mg, 4.90 mmol, 853.63 µL, 2 eq) was added to the mixture, and the mixture was stirred at 90 °C for 3 days. After cooling to room temperature, silica (1 g) was added to the mixture, and the solvent was removed under vacuum. The residue was purified using a silica column with petroleum ether:EtOAc=3:1→1:1 to obtain the compound of Formula H (61.94 mg, 2.3% yield) in a form of pale yellow oil.

¹H NMR (400 MHz, CHLOROFORM-*d*): *δ* 4.07-4.04 (m, 4H), 3.45 (td, 2H), 3.28 (s, 6H), 3.08 (br, 4H), 2.72 (br, H), 2.33-2.27 (m, 3H), 1.84 (br, 6H), 1.68-1.45 (m, 20H), 1.45-1.42 (m, 18H), 1.32-1.25 (m, 62H), 0.89 (t, 18H)

### Example 1: Preparation of composition for drug delivery using the lipid of Preparation Example 2 (the compound of Formula B) and test of drug delivery

### (1) Preparation of solutions for each component

The components shown in Table 1 below were dissolved in the respective dilution solvents and prepared at the concentrations shown in Table 1 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 1]**

| No. | Components | Concentration for use |
|---|---|---|
| 1 | mRNA | 1 mg/mL |
| 2 | Lipid of Preparation Example 2 (Compound of Formula B) | 10-20 mg/mL |
| 3 | DMG-PEG (1,2-dimyristoyl-rac-glycero-3-methoxypolyethylene glycol-2000) | 5-10 mg/mL |
| 4 | DOTMA (1,2-di-O-octadecenyl-3-trimethylammonium propane) | 10-20 mg/mL |
| 5 | Cholesterol | 10-20 mg/mL |

### (2) Mixing of raw materials

The components were taken and mixed according to the weight ratio of lipid of Preparation Example 2: DOTMA: cholesterol: DMG-PEG = 100:10.5:23.2:5.9, while maintaining an N/P ratio (amine groups of the lipid components/phosphate groups of the mRNA) of 5-10. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), the lipid of Preparation Example 2, DOTMA, cholesterol, and DMG-PEG were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 12 mL/min. When vortexing was used, the solution in Tube (B) was added to Tube (A) and mixed as rapidly and uniformly as possible to ensure formation of a homogeneous formulation.
6) The mixture obtained in step 5) was centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 2 below.

**[Table 2]**

| **No.** | **Lipid compound** | **NP ratio** | **Z-average (nm)** | **PDI (PI)** | **Zeta-potential (mV)** |
|---|---|---|---|---|---|
| 1 | Formula B | 5 (NP5) | 115.5 | 0.22 | 0.30 |
| 2 | Formula B | 10 (NP10) | 98.9 | 0.19 | -0.99 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 3 below.

**[Table 3]**

| Tissue | Protein Expression Levels of the Formulation of Example 1 in Mice | |
|---|---|---|
| | NP5 | NP10 |
| Lung | 7.12E+05 | 7.64E+05 |
| Liver | 1.06E+04 | 3.46E+04 |
| Spleen | 5.32E+04 | 3.90E+03 |
| Lung Expression Level Relative to Liver | 67-fold | 22-fold |

### Example 2: Preparation of composition for drug delivery using the lipid of Preparation Example 2 (the compound of Formula B) and test of drug delivery

### (1) Preparation of solutions for each component

The components shown in Table 4 below were dissolved in the respective dilution solvents and prepared at the concentrations shown in Table 4 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 4]**

| No. | Components | Concentration for use |
|---|---|---|
| 1 | mRNA | 1 mg/mL |
| 2 | Lipid of Preparation Example 2 (Compound of Formula B) | 10-20 mg/mL |
| 3 | mPEG-PLA(2K-4K) | 30-100 mg/mL |
| 4 | 18:0 Lyso PC (1-stearoyl-2-hydroxy-sn-glycero-3-phosphocholin) | 10-20 mg/mL |
| 5 | Cholesterol | 10-20 mg/mL |

| | | |
|---|---|---|
| mPEG-PLA(2K-4K): Copolymer of a monomethoxypolyethylene glycol (mPEG) block with a number average molecular weight of 2,000 and a polylactic acid (PLA) block with a number average molecular weight of 4,000 | | |

### (2) Mixing of raw materials

The components were taken and mixed according to the weight ratio of lipid of Preparation Example 2: 18:0 Lyso PC: cholesterol: mPEG-PLA(2K-4K) = 100:8.2:24.2:468.5, while maintaining an N/P ratio (amine groups of the lipid components/phosphate groups of the mRNA) of 5-10. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), mPEG-PLA(2K-4K), the lipid of Preparation Example 2, 18:0 Lyso PC, and cholesterol were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 3-12 mL/min. When vortexing was used, the solution in Tube (B) was added to Tube (A) and mixed as rapidly and uniformly as possible to ensure formation of a homogeneous formulation.
6) The mixture obtained in step 5) was diluted with DPBS and centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 5 below.

**[Table 5]**

| **No.** | **Lipid compound** | **NP ratio** | **Z-average (nm)** | **PDI (PI)** | **Zeta-potential (mV)** |
|---|---|---|---|---|---|
| 1 | Formula B | 5 (NP5) | 115.5 | 0.22 | 0.30 |
| 2 | Formula B | 10 (NP10) | 98.9 | 0.19 | -0.99 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 6 below.

**[Table 6]**

| Tissue | Protein Expression Levels of the Formulation of Example 2 in Mice | |
|---|---|---|
| | NP5 | NP10 |
| Lung | 1.52E+05 | 3.72E+05 |
| Liver | 6.83E+03 | 1.62E+03 |
| Spleen | 3.32E+04 | 1.02E+04 |
| Lung Expression Level Relative to Liver | 22-fold | 230-fold |

### Example 3: Preparation of composition for drug delivery using the lipid of Preparation Example 4 (the compound of Formula D) and test of drug delivery

### (1) Preparation of solutions for each component

The components shown in Table 7 below were dissolved in the respective dilution solvents and prepared at the concentrations shown in Table 7 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 7]**

| No. | Components | Concentration for use |
|---|---|---|
| 1 | mRNA | 1 mg/mL |
| 2 | Lipid of Preparation Example 4 (Compound of Formula D) | 10-20 mg/mL |
| 3 | DMG-PEG (1,2-dimyristoyl-rac-glycero-3-methoxypolyethylene glycol-2000) | 5-10 mg/mL |
| 4 | 18:0 Lyso PC (1-stearoyl-2-hydroxy-sn-glycero-3-phosphocholin) | 10-20 mg/mL |
| 5 | Cholesterol | 10-20 mg/mL |

### (2) Mixing of raw materials

The components were taken and mixed according to the weight ratio of lipid of Preparation Example 4: 18:0 Lyso PC: cholesterol: DMG-PEG = 100:8.8:24.9:6.3, while maintaining an N/P ratio (amine groups of the lipid components/phosphate groups of the mRNA) of 5-10. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), the lipid of Preparation Example 4, 18:0 Lyso PC, cholesterol, and DMG-PEG were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 12 mL/min. When vortexing was used, the solution in Tube (B) was added to Tube (A) and mixed as rapidly and uniformly as possible to ensure formation of a homogeneous formulation.
6) The mixture obtained in step 5) was centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 8 below.

**[Table 8]**

| **No.** | **Lipid compound** | **NP ratio** | **Z-average (nm)** | **PDI (PI)** | **Zeta-potential (mV)** |
|---|---|---|---|---|---|
| 1 | Formula D | 5 (NP5) | 136.4 | 0.18 | -2.25 |
| 2 | Formula D | 10 (NP10) | 117.0 | 0.17 | -0.82 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 9 below.

**[Table 9]**

| Tissue | Protein Expression Levels of the Formulation of Example 3 in Mice | |
|---|---|---|
| | NP5 | NP10 |
| Lung | 6.89E+05 | 7.04E+05 |
| Liver | 3.95E+04 | 4.88E+03 |
| Spleen | 1.47E+05 | 8.41E+04 |
| Lung Expression Level Relative to Liver | 17-fold | 144-fold |

### Example 4: Preparation of composition for drug delivery using the lipid of Preparation Example 4 (the compound of Formula D) and test of drug delivery

### (1) Preparation of solutions for each component

The components shown in Table 10 below were dissolved in the respective dilution solvents and prepared at the concentrations shown in Table 10 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 10]**

| No. | Components | Concentration for use |
|---|---|---|
| 1 | mRNA | 1 mg/mL |
| 2 | Lipid of Preparation Example 4 (Compound of Formula D) | 10-20 mg/mL |
| 3 | mPEG-PLA(2K-4K) | 30-100 mg/mL |
| 4 | DDAB (Dimethyldioctadecylammonium) | 10-20 mg/mL |
| 5 | Cholesterol | 10-20 mg/mL |

| | | |
|---|---|---|
| mPEG-PLA(2K-4K): Copolymer of a monomethoxypolyethylene glycol (mPEG) block with a number average molecular weight of 2,000 and a polylactic acid (PLA) block with a number average molecular weight of 4,000 | | |

### (2) Mixing of raw materials

The components were taken and mixed according to the weight ratio of lipid of Preparation Example 4: DDAB: cholesterol: mPEG-PLA(2K-4K) = 100:10.5:25.9:501.5, while maintaining an N/P ratio (amine groups of the lipid components/phosphate groups of the mRNA) of 5-10. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), mPEG-PLA(2K-4K), the lipid of Preparation Example 4, DDAB, and cholesterol were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 3-12 mL/min. When vortexing was used, the solution in Tube (B) was added to Tube (A) and mixed as rapidly and uniformly as possible to ensure formation of a homogeneous formulation.
6) The mixture obtained in step 5) was diluted with DPBS and centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 11 below.

**[Table 11]**

| **No.** | **Lipid compound** | **NP ratio** | **Z-average (nm)** | **PDI (PI)** | **Zeta-potential (mV)** |
|---|---|---|---|---|---|
| 1 | Formula D | 5 (NP5) | 112.5 | 0.16 | 4.43 |
| 2 | Formula D | 10 (NP10) | 106.4 | 0.19 | 10.89 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 12 below.

**[Table 12]**

| Tissue | Protein Expression Levels of the Formulation of Example 4 in Mice | |
|---|---|---|
| | NP5 | NP10 |
| Lung | 1.31E+06 | 1.97E+06 |
| Liver | 7.22E+03 | 9.86E+03 |
| Spleen | 3.44E+04 | 4.72E+04 |
| Lung Expression Level Relative to Liver | 181-fold | 200-fold |

### Example 5: Preparation of composition for drug delivery using the lipid of Preparation Example 5 (the compound of Formula F) and test of drug delivery

### (1) Preparation of solutions for each component

The components shown in Table 13 below were dissolved in the respective dilution solvents and prepared at the concentrations shown in Table 13 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 13]**

| No. | Components | Concentration for use |
|---|---|---|
| 1 | mRNA | 1 mg/mL |
| 2 | Lipid of Preparation Example 5 (Compound of Formula F) | 10-20 mg/mL |
| 3 | DMG-PEG (1,2-dimyristoyl-rac-glycero-3-methoxypolyethylene glycol-2000) | 5-10 mg/mL |
| 4 | DPPC (1,2-dipalmitoyl-sn-glycero-3-phosphocholine) | 10-20 mg/mL |
| 5 | Cholesterol | 10-20 mg/mL |

### (2) Mixing of raw materials

The components were taken and mixed according to the weight ratio of lipid of Preparation Example 5: DPPC: cholesterol: DMG-PEG = 100:9.9:20.1:5.1, while maintaining an N/P ratio (amine groups of the lipid components/phosphate groups of the mRNA) of 5-10. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), the lipid of Preparation Example 5, DPPC, cholesterol, and DMG-PEG were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 12 mL/min. When vortexing was used, the solution in Tube (B) was added to Tube (A) and mixed as rapidly and uniformly as possible to ensure formation of a homogeneous formulation.
6) The mixture obtained in step 5) was centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 14 below.

**[Table 14]**

| **No.** | **Lipid compound** | **NP ratio** | **Z-average (nm)** | **PDI (PI)** | **Zeta-potential (mV)** |
|---|---|---|---|---|---|
| 1 | Formula F | 5 (NP5) | 139.6 | 0.17 | -2.69 |
| 2 | Formula F | 10 (NP10) | 116.9 | 0.13 | -1.85 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 15 below.

**[Table 15]**

| Tissue | Protein Expression Levels of the Formulation of Example 5 in Mice | |
|---|---|---|
| | NP5 | NP10 |
| Lung | 1.18E+06 | 4.04E+06 |
| Liver | 2.45E+05 | 5.47E+05 |
| Spleen | 3.90E+05 | 2.05E+05 |
| Lung Expression Level Relative to Liver | 5-fold | 7-fold |

### Example 6: Preparation of composition for drug delivery using the lipid of Preparation Example 5 (the compound of Formula F) and test of drug delivery

### (1) Preparation of solutions for each component

The components shown in Table 16 below were dissolved in the respective dilution solvents and prepared at the concentrations shown in Table 16 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 16]**

| No. | Components | Concentration for use |
|---|---|---|
| 1 | mRNA | 1 mg/mL |
| 2 | Lipid of Preparation Example 5 (Compound of Formula F) | 10-20 mg/mL |
| 3 | mPEG-PLA(2K-4K) | 30-100 mg/mL |
| 4 | DDAB (Dimethyldioctadecylammonium) | 10-20 mg/mL |
| 5 | Cholesterol | 10-20 mg/mL |

| | | |
|---|---|---|
| mPEG-PLA(2K-4K): Copolymer of a monomethoxypolyethylene glycol (mPEG) block with a number average molecular weight of 2,000 and a polylactic acid (PLA) block with a number average molecular weight of 4,000 | | |

### (2) Mixing of raw materials

The components were taken and mixed according to the weight ratio of lipid of Preparation Example 5: DDAB: cholesterol: mPEG-PLA(2K-4K) = 100:8.5:20.9:405.7, while maintaining an N/P ratio (amine groups of the lipid components/phosphate groups of the mRNA) of 5-10. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), mPEG-PLA(2K-4K), the lipid of Preparation Example 5, DDAB, and cholesterol were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 3-12 mL/min. When vortexing was used, the solution in Tube (B) was added to Tube (A) and mixed as rapidly and uniformly as possible to ensure formation of a homogeneous formulation.
6) The mixture obtained in step 5) was diluted with DPBS and centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 17 below.

**[Table 17]**

| **No.** | **Lipid compound** | **NP ratio** | **Z-average (nm)** | **PDI (PI)** | **Zeta-potential (mV)** |
|---|---|---|---|---|---|
| 1 | Formula F | 5 (NP5) | 127.6 | 0.13 | -3.08 |
| 2 | Formula F | 10 (NP10) | 161.8 | 0.11 | -2.86 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 18 below.

**[Table 18]**

| Tissue | Protein Expression Levels of the Formulation of Example 6 in Mice | |
|---|---|---|
| | NP5 | NP10 |
| Lung | 3.80E+06 | 2.56E+07 |
| Liver | 1.98E+04 | 4.89E+04 |
| Spleen | 1.30E+05 | 5.85E+05 |
| Lung Expression Level Relative to Liver | 192-fold | 524-fold |

### Example 7: Preparation of composition for drug delivery using the lipid of Preparation Example 7 (the compound of Formula H) and test of drug delivery

### (1) Preparation of solutions for each component

The components shown in Table 19 below were dissolved in the respective dilution solvents and prepared at the concentrations shown in Table 19 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 19]**

| No. | Components | Concentration for use |
|---|---|---|
| 1 | mRNA | 1 mg/mL |
| 2 | Lipid of Preparation Example 7 (Compound of Formula H) | 10-20 mg/mL |
| 3 | DMG-PEG (1,2-dimyristoyl-rac-glycero-3-methoxypolyethylene glycol-2000) | 5-10 mg/mL |
| 4 | DSPC(1,2-distearoyl-sn-glycero-3-phosphocholine) | 10-20 mg/mL |
| 5 | Cholesterol | 10-20 mg/mL |

### (2) Mixing of raw materials

The components were taken and mixed according to the weight ratio of lipid of Preparation Example 7: DSPC: cholesterol: DMG-PEG = 100:14.1:26.6:6.7, while maintaining an N/P ratio (amine groups of the lipid components/phosphate groups of the mRNA) of 5-20. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), the lipid of Preparation Example 7, DSPC, cholesterol, and DMG-PEG were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 12 mL/min. When vortexing was used, the solution in Tube (B) was added to Tube (A) and mixed as rapidly and uniformly as possible to ensure formation of a homogeneous formulation.
6) The mixture obtained in step 5) was centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 20 below.

**[Table 20]**

| **No.** | **Lipid compound** | **NP ratio** | **Z-average (nm)** | **PDI (PI)** | **Zeta-potential (mV)** |
|---|---|---|---|---|---|
| 1 | Formula H | 5 (NP5) | 141.1 | 0.16 | -2.56 |
| 2 | Formula H | 10 (NP10) | 124.9 | 0.17 | -1.80 |
| 3 | Formula H | 20 (NP20) | 96.8 | 0.19 | -1.25 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 21 below.

**[Table 21]**

| Tissue | Protein Expression Levels of the Formulation of Example 7 in Mice | | |
|---|---|---|---|
| | NP5 | NP10 | NP20 |
| Lung | 4.24E+05 | 7.42E+05 | 1.41E+06 |
| Liver | 3.76E+04 | 8.08E+04 | 3.59E+04 |
| Spleen | 1.33E+05 | 1.22E+05 | 9.42+04 |
| Lung Expression Level Relative to Liver | 11-fold | 9-fold | 39-fold |

### Example 8: Preparation of composition for drug delivery using the lipid of Preparation Example 7 (the compound of Formula H) and test of drug delivery

### (1) Preparation of solutions for each component

The components shown in Table 22 below were dissolved in the respective dilution solvents and prepared at the concentrations shown in Table 22 below. During dissolution, the materials were brought to room temperature, the solvent was added, and the materials were dissolved to the desired concentrations. After confirming by visual inspection that no undissolved particles remained, the resulting solutions were used for preparation of the compositions.

**[Table 22]**

| No. | Components | Concentration for use |
|---|---|---|
| 1 | mRNA | 1 mg/mL |
| 2 | Lipid of Preparation Example 7 (Compound of Formula H) | 10-20 mg/mL |
| 3 | mPEG-PLA(2K-4K) | 30-100 mg/mL |
| 4 | DOPE(dioleoyl-phosphatidyl-ethanolamine) | 10-20 mg/mL |
| 5 | Cholesterol | 10-20 mg/mL |

| | | |
|---|---|---|
| mPEG-PLA(2K-4K): Copolymer of a monomethoxypolyethylene glycol (mPEG) block with a number average molecular weight of 2,000 and a polylactic acid (PLA) block with a number average molecular weight of 4,000 | | |

### (2) Mixing of raw materials

The components were taken and mixed according to the weight ratio of lipid of Preparation Example 7: DOPE: cholesterol: mPEG-PLA(2K-4K) = 100:13.3:27.6:536.2, while maintaining an N/P ratio (amine groups of the lipid components/phosphate groups of the mRNA) of 5-20. Ethanol was additionally added to the ethanol phase such that the molecular total of all components was 6.25-12.5 mM, and the mRNA was diluted in the aqueous phase using 20 mM sodium acetate buffer (pH 4.6). The aqueous phase and the ethanol phase were mixed while maintaining a ratio of 3:1. After mixing, the mixture was diluted with DPBS (Dulbecco's phosphate-buffered saline) such that the ethanol concentration was 5% or less in order to reduce the total ethanol content, and was then subjected to buffer exchange and concentration as follows: the mixture was buffer-exchanged through dilution and concentration by centrifugation at 4,000 rpm using an Amicon-Ultra tube filter (Merck Millipore, UFC505096 or UFC805024, pore size: 50K or 100K, volume: 0.5 mL, 4 mL, or 15 mL).

The specific process was carried out as follows:
1) Two autoclaved tubes were prepared (Tubes (A) and (B)).
2) To Tube (A), mPEG-PLA(2K-4K), the lipid of Preparation Example 7, DOPE, and cholesterol were sequentially added in amounts corresponding to the numbers of moles calculated according to the experimental conditions, and mixed by vortexing.
3) Ethanol was added to the ethanol phase, as necessary, such that the total molar concentration of all components was within 6.25-12.5 mM.
4) mRNA and 20 mM sodium acetate buffer (pH 4.6) were mixed in Tube (B). At this time, the components were added such that the volume of the aqueous phase was three times the total volume of the ethanol phase.
5) The contents of Tube (A) and Tube (B) were mixed using a microfluidics device (Ignite, Precision Nanosystems) or by vortexing. The microfluidic operating conditions were a flow rate ratio (FRR) of C:R = 3:1 and a total flow rate (TRR) of 3-12 mL/min. When vortexing was used, the solution in Tube (B) was added to Tube (A) and mixed as rapidly and uniformly as possible to ensure formation of a homogeneous formulation.
6) The mixture obtained in step 5) was diluted with DPBS and centrifuged at 4,000 rpm using an Amicon-Ultra tube filter (50K or 100K), and the concentration and dilution processes were repeated to remove excess ethanol until the ethanol content was 0.1% or less, after which the mixture was concentrated to the final target concentration.
7) After the desired concentration was reached, the formulation was sterilized using a 0.22 µm pore size filter.
8) The concentration was measured using a Ribo-green assay prior to use.

### (3) Evaluation of Formulation Properties

The particle characteristics of the prepared formulations were analyzed using a particle size analyzer based on dynamic light scattering (DLS), and the results are shown in Table 23 below.

**[Table 23]**

| **No.** | **Lipid compound** | **NP ratio** | **Z-average (nm)** | **PDI (PI)** | **Zeta-potential (mV)** |
|---|---|---|---|---|---|
| 1 | Formula H | 5 (NP5) | 108.0 | 0.15 | -3.70 |
| 2 | Formula H | 10 (NP10) | 94.4 | 0.19 | -0.42 |
| 3 | Formula H | 20 (NP20) | 86.6 | 0.20 | -0.54 |

### (4) Administration of composition

The prepared formulations were adjusted to a concentration of 10 µg/mL and intravenously administered to mice such that 2 µg of mRNA was administered per mouse. Four hours after administration, protein expression in the whole body and in individual organs was evaluated using an IVIS Spectrum in vivo imaging system, and the results are shown in Table 24 below.

**[Table 24]**

| Tissue | Protein Expression Levels of the Formulation of Example 8 in Mice | | |
|---|---|---|---|
| | NP5 | NP10 | NP20 |
| Lung | 1.45E+05 | 1.38E+05 | 5.31E+06 |
| Liver | 5.57E+03 | 1.29E+03 | 2.75E+03 |
| Spleen | 3.53E+04 | 1.21E+04 | 1.36E+04 |
| Lung Expression Level Relative to Liver | 26-fold | 107-fold | 1931-fold |

## Claims

1. A composition for drug delivery comprising:
effective ingredient selected from nucleic acid, polypeptide, virus or combination thereof;
a cationic lipid having a structure represented by the following Formula 1; and
lipid-polymer, amphiphilic block copolymer, or a combination thereof: 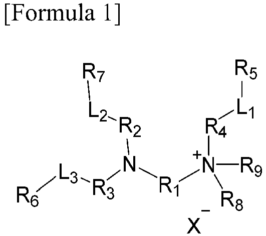
wherein, in the above Formula 1,
R₁ is a substituted or unsubstituted alkylene group, alkenylene group or alkynylene group,
each of R₂, R₃ and R₄ is independently a substituted or unsubstituted alkylene group, alkenylene group or alkynylene group,
each of R₅, R₆ and R₇ is independently a substituted or unsubstituted, saturated or unsaturated monovalent hydrocarbon group,
each of R₈ and R₉ is independently a substituted or unsubstituted alkyl group, alkenyl group, alkynyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, alkynylene, arylene, and heteroarylene, wherein L' is a direct bond, alkylene, alkenylene or alkynylene, and each R' is independently selected from the group consisting of hydrogen atom, alkyl, alkenyl and alkynyl,
n is 0 or 1, and
X⁻ is a pharmaceutically acceptable monovalent anion.

2. The composition for drug delivery according to claim 1, wherein
R₁ is a substituted or unsubstituted C₁₋₆ alkylene group, C₂₋₆ alkenylene group or C₂₋₆ alkynylene group,
each of R₂, R₃ and R₄ is independently a substituted or unsubstituted C₃₋₁₂ alkylene group, C₃₋₁₂ alkenylene group or C₃₋₁₂ alkynylene group,
each of R₅, R₆ and R₇ is independently a substituted or unsubstituted, saturated or unsaturated monovalent C₃₋₂₀ hydrocarbon group,
each of R₈ and R₉ is independently a substituted or unsubstituted C₁₋₆ alkyl group, C₂₋₆ alkenyl group, C₂₋₆ alkynyl group or C₃₋₆ carbocyclic group, or is independently -R₁₀-(L₄)ₙ-R₁₁,
each R₁₀ is independently a substituted or unsubstituted C₃₋₁₂ alkylene group,
each R₁₁ may be independently a substituted or unsubstituted, saturated or unsaturated monovalent C₃₋₂₀ hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, C₂₋₆ alkenylene, C₂₋₆ alkynylene, C₆₋₂₀ arylene, and C₃₋₂₀ heteroarylene, wherein L' is a direct bond, C₁₋₁₃ alkylene, C₂₋₁₃ alkenylene or C₂₋₁₃ alkynylene, and each R' is independently selected from the group consisting of hydrogen atom, C₁₋₁₈ alkyl, C₂₋₁₈ alkenyl and C₂₋₁₈ alkynyl,
n is 0 or 1, and
X⁻ is a pharmaceutically acceptable monovalent anion of inorganic acid or organic acid.

3. The composition for drug delivery according to claim 1, wherein
R₁ is a substituted or unsubstituted C₃₋₄ alkylene group, C₃₋₄ alkenylene group or C₃₋₄ alkynylene group,
each of R₂, R₃ and R₄ is independently a substituted or unsubstituted C₆₋₈ alkylene group, C₆₋₈ alkenylene group or C₆₋₈ alkynylene group,
each of R₅, R₆ and R₇ is independently a substituted or unsubstituted, saturated or unsaturated monovalent C₅₋₁₅ hydrocarbon group,
each of R₈ and R₉ is independently a substituted or unsubstituted C₁₋₂ alkyl group, C₂₋₃ alkenyl group or C₂₋₃ alkynyl group, or is independently -R₁₀-(L₄)ₙ-R₁₁,
each R₁₀ is independently a substituted or unsubstituted C₆₋₈ alkylene group,
each R₁₁ is independently a substituted or unsubstituted, saturated or unsaturated monovalent C₅₋₁₅ hydrocarbon group,
each of L₁, L₂, L₃ and L₄ is independently selected from the group consisting of -C(O)O-, -OC(O)-, -C(O)N(R')-, -N(R')C(O)-, -P(O)(OR')O-, -S-S-, C₂₋₅ alkenylene and C₂₋₅ alkynylene, wherein each R' is independently selected from the group consisting of hydrogen atom, C₁₋₆ alkyl, C₂₋₆ alkenyl and C₂₋₆ alkynyl,
n is 0 or 1, and
X⁻ is halide, nitrate anion (NO₃⁻), benzoate anion (C₆H₅COO⁻), methanesulfonate anion, acetate anion (CH₃COO⁻) (i.e., AcO⁻), or trihaloacetate anion (CF₃COO⁻).

4. The composition for drug delivery according to claim 1, wherein
R₁ is a substituted or unsubstituted C₃₋₄ alkylene group,
each of R₂, R₃ and R₄ is independently a substituted or unsubstituted C₆₋₈ alkylene group,
each of R₅, R₆ and R₇ is independently a substituted or unsubstituted, saturated or unsaturated monovalent C₅₋₁₅ hydrocarbon group,
each of R₈ and R₉ is independently a substituted or unsubstituted C₁₋₂ alkyl group, or is independently -R₁₀-(L₄)ₙ-R₁₁,
each R₁₀ is independently a substituted or unsubstituted C₆₋₈ alkylene group,
each R₁₁ is independently a substituted or unsubstituted, saturated or unsaturated monovalent C₅₋₁₅ hydrocarbon group,
each of L₁, L₂, L₃ and L₄ is independently selected from the group consisting of -C(O)O-, -OC(O)-, -C(O)N(R')-, -N(R')C(O)-, -P(O)(OR')O-, -S-S-, and C₂₋₅ alkenylene, wherein each R' is independently selected from the group consisting of hydrogen atom and C₁₋₆ alkyl,
n is 0 or 1, and
X⁻ is Cl⁻, Br⁻, or acetate anion (CH₃COO⁻) (i.e., AcO⁻).

5. The composition for drug delivery according to claim 1, wherein the cationic lipid is one having a structure selected from the following Formulas A to Q:
| Formula | Structure |
|---|---|
| A | 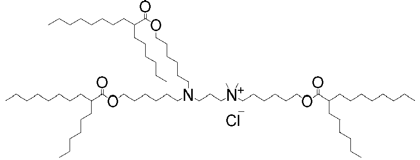 |
| B | 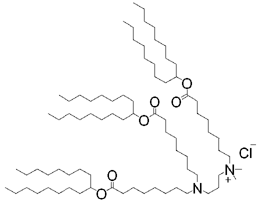 |
| C | 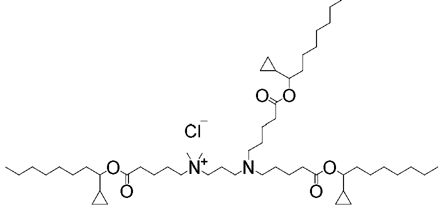 |
| D | 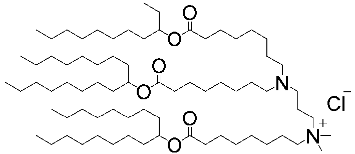 |
| E | 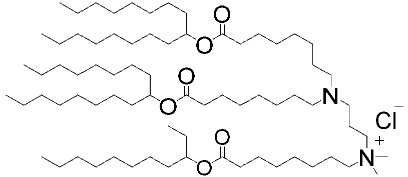 |
| F | 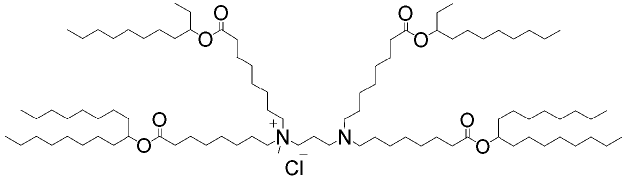 |
| G | 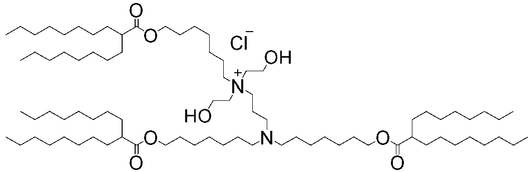 |
| H | 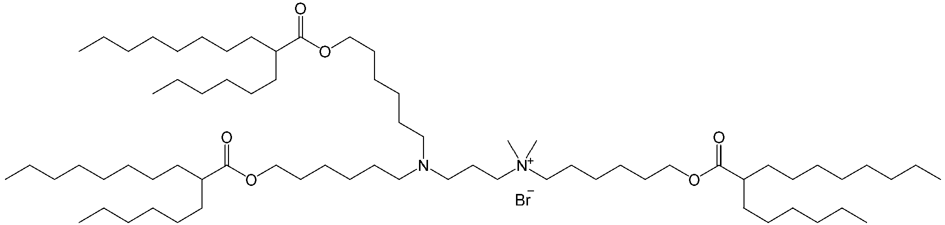 |
| I | 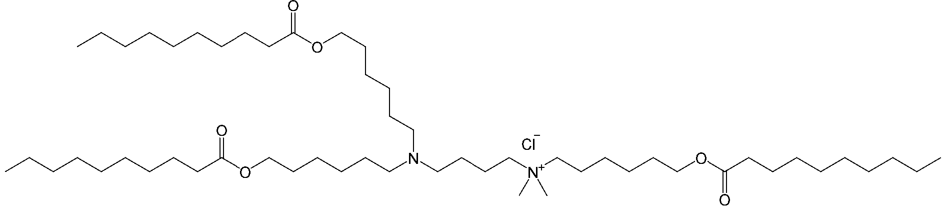 |
| J | 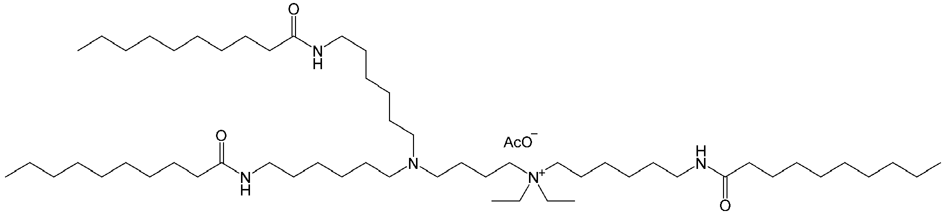 |
| K | 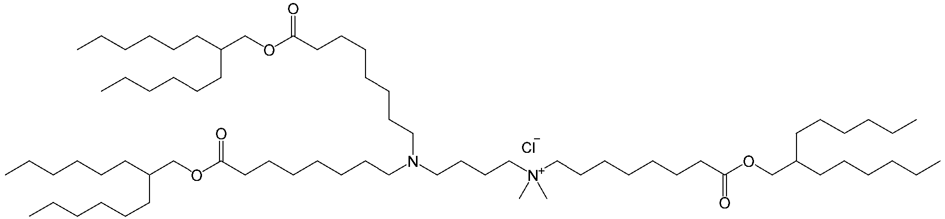 |
| L | 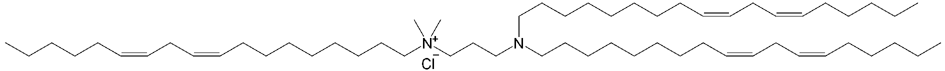 |
| M | 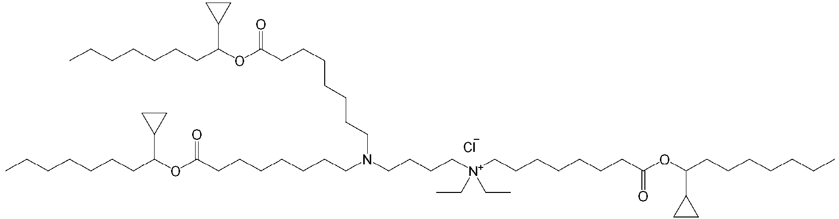 |
| N | 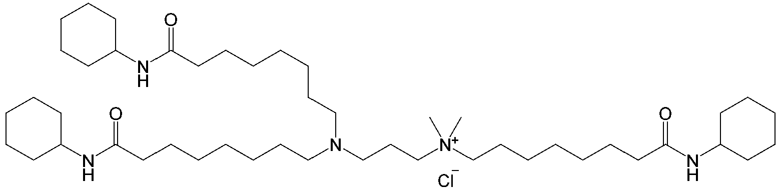 |
| O | 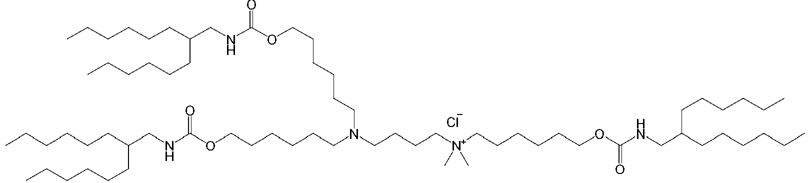 |
| P | 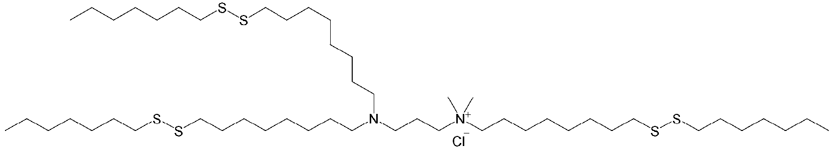 |
| Q | 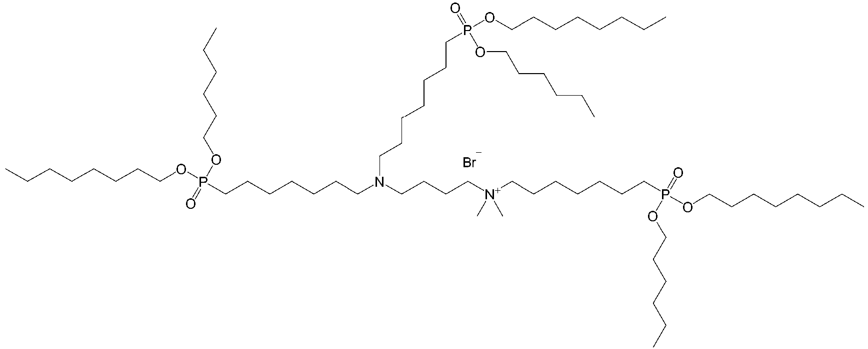 |

6. The composition for drug delivery according to claim 1, wherein the effective ingredient is mRNA.

7. The composition for drug delivery according to claim 1, wherein the lipid-polymer is a polymer in which one or more saturated or unsaturated hydrocarbon groups having 11 to 25 carbon atoms as a hydrophobic part are introduced into a hydrophilic block which is a hydrophilic part.

8. The composition for drug delivery according to claim 7, wherein the hydrophilic block is one or more selected from the group consisting of polyalkylene glycol, polyvinyl alcohol, polyvinyl pyrrolidone, polyacrylamide, and derivatives thereof.

9. The composition for drug delivery according to claim 7, wherein the saturated or unsaturated hydrocarbon group having 11 to 25 carbon atoms is independently selected from the group consisting of lauryl, myristoyl, dimyristoyl, myristyl, palmityl, stearyl, arachidyl, behenyl, lignoceryl, cerotyl, myristoleyl, palmitoleyl, sapienyl, oleyl, linoleyl, arachidonyl, eicosapentaenyl, erucyl, and docosahexaenyl.

10. The composition for drug delivery according to claim 1, wherein the amphiphilic block copolymer is an A-B type block copolymer comprising a hydrophilic A block and a hydrophobic B block, wherein the hydrophilic A block is one or more selected from the group consisting of polyalkylene glycol, polyvinyl alcohol, polyvinyl pyrrolidone, polyacrylamide, and derivatives thereof, and the hydrophobic B block is one or more selected from the group consisting of polyester, polyanhydride, polyamino acid, polyorthoester and polyphosphazine.

11. The composition for drug delivery according to claim 10, wherein the hydroxyl group at the end of the hydrophobic B block is modified with one or more selected from the group consisting of cholesterol, tocopherol, and fatty acids having 10 to 24 carbons.

12. The composition for drug delivery according to any one of claims 1 to 11, wherein the composition further comprises fusogenic lipid.

13. The composition for drug delivery according to claim 12, wherein the fusogenic lipid is one or a combination of two or more selected from the group consisting of phospholipid, cholesterol, and tocopherol.

14. A method for preparing a composition for drug delivery, comprising the steps of:
(a) preparing a solution in which a cationic lipid represented by the following Formula 1; and lipid-polymer, amphiphilic block copolymer, or a mixture thereof; are dissolved in a water-miscible organic solvent; and
(b) to the solution prepared in step (a), adding effective ingredient selected from nucleic acid, polypeptide, virus, or combination thereof, and mixing them: 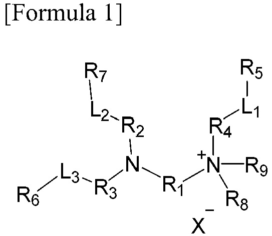 wherein, in the above Formula 1,
R₁ is a substituted or unsubstituted alkylene group, alkenylene group or alkynylene group,
each of R₂, R₃ and R₄ is independently a substituted or unsubstituted alkylene group, alkenylene group or alkynylene group,
each of R₅, R₆ and R₇ is independently a substituted or unsubstituted, saturated or unsaturated monovalent hydrocarbon group,
each of R₈ and R₉ is independently a substituted or unsubstituted alkyl group, alkenyl group, alkynyl group or carbocyclic group, or is independently -R₁₀-(L₄)ₙ-R₁₁,
each R₁₀ is independently a substituted or unsubstituted alkylene group,
each R₁₁ is independently a substituted or unsubstituted, saturated or unsaturated monovalent hydrocarbon group,
each of L₁, L₂, L₃ and L₄ is independently selected from the group consisting of -C(O)O-, -OC(O)-, -OC(O)-L'-C(O)O-, -C(O)N(R')-, -N(R')C(O)-, -C(O)-, -C(S)-, -C(S)S-, -SC(S)-, - CH(OH)-, -P(O)(OR')O-, -S(O)₂-, -S-S-, alkenylene, alkynylene, arylene, and heteroarylene, wherein L' is a direct bond, alkylene, alkenylene or alkynylene, and each R' is independently selected from the group consisting of hydrogen atom, alkyl, alkenyl and alkynyl,
n is 0 or 1, and
X⁻ is a pharmaceutically acceptable monovalent anion.

15. The method for preparing a composition for drug delivery according to claim 14, wherein the water-miscible organic solvent in step (a) is ethanol.

16. The method for preparing a composition for drug delivery according to claim 14, wherein step (b) comprises:
(b-1) a step of preparing a buffer solution containing the effective ingredient; and
(b-2) a step of adding the buffer solution of the effective ingredient prepared in step (b-1) to the solution prepared in step (a), and mixing them.

17. The method for preparing a composition for drug delivery according to claim 14, wherein step (b) comprises:
(b-1) a step of adding the effective ingredient to the solution prepared in step (a); and
(b-2) a step of adding a buffer solution to the resulting mixture of step (b-1) and mixing them.
